# EUROPEAN PATENT SPECIFICATION

(11) **EP 2 158 325 B1**
(45) Date of publication and mention of the grant of the patent: **25.05.2016**
(21) Application number: 08771410.1
(22) Date of filing: 18.06.2008
(51) Int. Cl.: C12P 19/34

(54) **METHODS FOR NUCLEIC ACID AMPLIFICATION**
VERFAHREN ZUR NUKLEINSÄUREAMPLIFIKATION
PROCÉDÉS POUR L'AMPLIFICATION D'ACIDES NUCLÉIQUES

(30) Priority: 18.06.2007 US 944708 P; 16.06.2008 US 140142
(43) Date of publication of application: 03.03.2010
(73) Proprietor: Life Technologies Corporation, Carlsbad, CA 92008 (US)
(72) Inventor: MULERO, Julio J., Sunnyvale, California 94086 (US); HENNESSY, Lori K., San Mateo, California 94403 (US)
(74) Representative: Grund, Martin
(86) International application number: PCT/US2008/067403
(87) International publication number: WO 2008/157641

(56) References cited:
- US-A1- 2003 044 817
- US-A1- 2005 037 361
- US-A1- 2005 112 591
- US-A1- 2006 014 190
- US-A1- 2006 147 924
- US-A1- 2006 252 077
- US-A1- 2007 037 182
- US-A1- 2007 037 182
- US-B1- 6 395 486
- US-B1- 7 008 771
- COLLINS P J ET AL: "DEVELOPMENTAL VALIDATION OF A SINGLE-TUBE AMPLIFICATION OF THE 13 CODIS STR LOCI, D2S1338, D19S433, AND AMELOGENIN: THE AMPFLSTR IDENTIFILER PCR AMPLIFICATION KIT", JOURNAL OF FORENSIC SCIENCES, CALLAGHAN AND CO, CHICAGO, IL, US, vol. 49, no. 6, 1 November 2004 (2004-11-01), pages 1265-1277, XP008054204, ISSN: 0022-1198, DOI: 10.1520/JFS2002195
- CAROLYN R. HILL ET AL: "Concordance Study Between the AmpF?STR � MiniFiler TM PCR Amplification Kit and Conventional STR Typing Kits", JOURNAL OF FORENSIC SCIENCES, vol. 52, no. 4, 6 June 2007 (2007-06-06), pages 870-873, XP55010510, ISSN: 0022-1198, DOI: 10.1111/j.1556-4029.2007.00491.x [retrieved on 2007-06-06]
- BUTLER J M ET AL: "THE DEVELOPMENT OF REDUCED SIZE STR AMPLICONS AS TOOLS FOR ANALYSIS OF DEGRADED DNA", JOURNAL OF FORENSIC SCIENCES, CALLAGHAN AND CO, CHICAGO, IL, US, vol. 48, no. 5, 1 September 2003 (2003-09-01), pages 1054-1064, XP008065803, ISSN: 0022-1198
- MULERO JULIO J ET AL: "Development and validation of the AmpFlSTR MiniFiler PCR Amplification Kit: a MiniSTR multiplex for the analysis of degraded and/or PCR inhibited DNA.", JOURNAL OF FORENSIC SCIENCES JUL 2008 LNKD- PUBMED:18540972, vol. 53, no. 4, July 2008 (2008-07), pages 838-852, XP002662367, ISSN: 1556-4029
- GROSSMAN ET AL: "HIGH-DENSITY MULTIPLEX DETECTION OF NUCLEIC ACID SEQUENCES: OLIGONUCLEOTIDE LIGATION ASSAY AND SEQUENCE-CODED SEPARATION", NUCLEIC ACIDS RESEARCH, OXFORD : OXFORD UNIV. PRESS, 1974-ANFANGS: LONDON : INFORMATION RETRIEVAL LTD, GB, vol. 22, no. 21, 1 January 1994 (1994-01-01), pages 4527-4534, XP002144258, ISSN: 0305-1048
- KAUPPINEN ET AL: "Locked nucleic acid (LNA): High affinity targeting of RNA for diagnostics and therapeutics", DRUG DISCOVERY TODAY: TECHNOLOGIES, ELSEVIER, AMSTERDAM, NL, vol. 2, no. 3, 1 October 2005 (2005-10-01) , pages 287-290, XP005118470, ISSN: 1740-6749, DOI: 10.1016/J.DDTEC.2005.08.012
- DI GIUSTO DANIEL A ET AL: 'Strong positional preference in the interaction of LNA oligonucleotides with DNA polymerase and proofreading exonuclease activities: implications for genotyping assays' NUCLEIC ACIDS RESEARCH, OXFORD UNIVERSITY PRESS, GB vol. 32, no. 3, E32, 01 January 2004, pages 1 - 8, XP002424243 DOI: 10.1093/NAR/GNH036 ISSN: 0305-1048
- SUTLOVIC DAVORKA ET AL: "Taq polymerase reverses inhibition of quantitative real time polymerase chain reaction by humic acid", CROATIAN MEDICAL JOURNAL, vol. 46, no. 4, August 2005 (2005-08), pages 556-562, ISSN: 0353-9504

## Description

### Cross-Reference to Related Applications

This application claims a priority benefit under 35 U.S. C. $ 119(e) from. U.S. Patent Application No. 60/944,708, filed June 18, 2007.

### Field

The present disclosure relates to methods and use of kits for nucleic acid sequence amplification.

### Introduction

Polymerase chain reaction (PCR) amplification of DNA samples from crime scenes or other non-sterile environments can be affected by inhibitors present in the samples themselves. For example, outdoor crimes may leave body fluids such as blood and semen deposited on soil, sand, or wood which can contain substances that could co- extract with the sample's DNA and prevent PCR amplification. Textile dyes, leather, and wood from interior crime scenes may also contain DNA polymerase inhibitors. The end result of amplifying a DNA sample containing an inhibitor may be a partial or even complete loss of alleles in a short tandem repeat (STR) multiplex.

US 2007/037182 A1 discloses oligonucleotide primers and probes for detecting ancestry informative marker (AIMs) polynucleotides that contain a single nucleotide polymorphism or that contain an insertion or deletion, as are methods of using the oligonucleotide primers and probes to draw an inference as to a trait of an individual. The trait can be, for example, biogeographical ancestry, a pigmentation trait, responsiveness to a drug, or susceptibility to a disease. Also provided are methods of determining the proportional ancestry of an individual. Reagents and kits also are provided.

From US 2006/014190 A1 methods for genotyping a sample comprising nucleic acid are known. The methods comprise analyzing STR and SNP loci.

Collins P.J. et al., Journal of Forensic Sciences (2004) 1265-1277 relates to developmental validation of a single tube amplification of the 13 codies STR loci, D2S1338, D19S433 and amelogenin.

US 2005/112591 A1 teaches methods of purifying, isolating, separating, and identifying target nucleic acids. In some embodiments an affinity moiety can be incorporated into one of the flanking primers of a nucleic acid amplification reaction primer pair. The reaction mixture can be contacted with a binding moiety specific for the affinity moiety, thereby allowing immobilization of the double stranded amplification product, separation of reaction components lacking the affinity moiety, and isolation of the target nucleic acid strand. Further, one of the flanking primers of the nucleic acid amplification reaction can comprise a label and a mobility modifier, thereby facilitating identification of the target nucleic acids. In some embodiments, the amplification reaction is multiplexed and comprises polymorphic microsatellites useful in human identification. and the manufacturing of molecular size standards. Some embodiments of the present teachings provide for improved methods of performing electrokinetic injection.

Hill C.R. et al., Journal of Forensic Sciences (2007) 870-873 concerns a concordance study between AmpF?STR.

US 7 008 771 B1 discloses methods and materials for use in simultaneously amplifying at least thirteen loci of genomic DNA in a single multiplex reaction, as are methods and materials for use in the analysis of the products of such reactions. Included are materials and methods for the simultaneous amplification of at least thirteen short tandem repeat loci, including specific materials and methods for the analysis of thirteen such loci specifically selected by the United States Federal Bureau of Investigation as core loci for use in the Combined DNA Index System (CODIS) database.

US 2003/044817 A1 provides modified primers for use in the amplification of a nucleic acid sequence. Amplifications carried out using the modified primers result in less template-independent non-specific product (primer dimer) compared to amplifications carried out using unmodified primers.

### SUMMARY

In some aspect, a method is provided, comprising contacting a sample comprising a nucleic acid and a PCR inhibitor that is humic acid with a primer, wherein the primer comprises a single high stability nucleic acid analog, wherein the single high stability nucleic acid analog is an LNA, and wherein the primer exhibits a higher degree of amplification ability in the presence of humic acid than a standard primer not comprising said single LNA; and performing a PCR amplification reaction on the sample, wherein a target nucleic acid sequence is amplified by PCR with the primer comprising said single LNA.

The PCR amplification with the primer can amplify a nucleic acid sequence from at least one locus selected from the group consisting of: CSF1PO, FGA, TH01, TPOX, vWA, D3S1358, DSS818, D7S820, DSS1179, D13S317, D16S539, D18S51, D21S11, D19S433, D2S1338, and amelogenin. The primer can comprise a sequence that allows the amplification of a short tandem repeat. The primer can further comprise a mobility modifier. The mobility modifier can be selected from the group consisting of: polyethylene oxide, polyglycolic acid, polylactic acid, polypeptide, oligosaccharide, polyurethane, polyamide, polysulfonamide, polysulfoxide, polyphosphonate, and block copolymers thereof.

In some aspect, the use of a kit for performing the above method is provided, the kit comprising: deoxynucleotide triphosphate; a fluorescently labeled primer; a high stability primer comprising a single high stability nucleic acid analog, wherein the single high stability nucleic acid analog is LNA, and wherein the primer exhibits a higher degree of amplification ability in the presence of humic acid than a standard primer not comprising said single LNA; a DNA polymerase; and optionally an allelic ladder corresponding to sizes that are appropriate for comparison to a short tandem repeat analysis.

### Drawings

FIG. 1 shows certain exemplary embodiments of amplifying a target nucleic acid sequence.
FIG. 2 shows certain exemplary embodiments of amplifying a target nucleic acid sequence.
FIG. 3A shows certain exemplary embodiments of amplifying a target nucleic acid sequence.
FIG. 3B shows certain exemplary embodiments of amplifying a target nucleic acid sequence.
FIG. 4 shows performance of Amelogenin (Amel)-LNA oligos with or without humic acid.

The skilled artisan will understand that the drawings are provided for illustration purposes only. The drawings are not intended to limit the scope of the present teachings in any way.

### Description Of Various Embodiments

Some embodiments of the present teachings provide compositions and methods that facilitate nucleic acid amplification. In some embodiments, the present teachings provide methods and compositions for overcoming or reducing amplification inhibition, especially for compositions that, at some point, are located in a non-sterile environment and can contain contaminants, such as PCR inhibitors. In some embodiments, the present teachings provide methods and compositions for the enhanced robustness of existing amplification protocols, even when such. PCR inhibitors are present.

As will be appreciated by one of skill in the art, while general nucleic acid amplification in a laboratory can be routine to one of skill in the art, the ability to amplify nucleic acid samples that are from non-laboratory conditions, such as samples taken from a crime scene, can include nucleic acid amplification inhibitors. One way to reduce the impact of PCR inhibitors is to select another priming sequence, in the same gene or locus. However, the present Inventors have realized that such an approach has many potential downsides (*e.g.,* where certain primers have already been established as acceptable and a substantial amount of work is required to introduce new primers). Some embodiments disclosed in the present disclosure demonstrate how this potential downside can be resolved. Rather than selecting a new primer, it has been discovered that there are substantial benefits to keeping the same basic primer sequence and modifying it with a high stability nucleic acid analog so that the primer displays a higher stability when hybridized (*e.g.,* it has a greater melting point than a comparable primer). As the substitution of the high stability nucleic acid analog can be comparable, a high amount of guidance is provided in regard to how each primer should be modified to achieve the desired results. Additionally, this approach can readily be applied to kits, compositions, and techniques that already have characterized primers (*e.g.,* which can be used to amplify STR markers, including the CODIS loci).

As will be appreciated by one of skill in the art, in light of the present disclosure, preserving original primer sequences in modified high stability primers can be important because it ensures genotypic concordance with the original unmodified primer. An advantage of maintaining the same primer sequences is to prevent allele dropout due to primer binding site mutations or polymorphisms.

Information obtained from amplifying a target nucleic acid sequence in a sample can be used in various applications. For example, the information can be used in genetic mapping, linkage analysis, clinical diagnostics, or identity testing. In some embodiments, the information can be used to identify the source (*e.g.,* a target individual), or narrow down the possible sources, of the nucleic acid. In certain such embodiments, the information can be used, *e.g.,* in forensic identification, paternity testing, DNA profiling, and related applications.

### Some Definitions

The term "nucleotide base," as used herein, refers to a substituted or unsubstituted aromatic ring or rings. In some embodiments, the aromatic ring or rings contain at least one nitrogen atom. In some embodiments, the nucleotide base is capable of forming Watson-Crick and/or Hoogsteen hydrogen bonds with an appropriately complementary nucleotide base. Exemplary nucleotide bases and analogs thereof include, but are not limited to, naturally occurring nucleotide bases adenine, guanine, cytosine, 6 methyl-cytosine, uracil, thymine, and analogs of the naturally occurring nucleotide bases, *e.g.,* 7-deazaadenine, 7-deazaguanine, 7-deaza-8-azaguanine, 7-deaza-8-azaadenine, N6 - Δ2 -isopentenyladenine (6iA), N6 -Δ2-isopentenyl-2-methylthioadenine (2ms6iΔ), N2 - dimethylguanine (dmG), 7-methylguanine (7mG), inosine, nebularine, 2-aminopurine, 2-amino-6-chloropurine, 2,6-diaminopurine, hypoxanthine, pseudouridine, pseudocytosine, pseudoisocytosine, 5-propynylcytosine, isocytosine, isoguanine, 7-deazaguanine, 2-thiopyrimidine, 6-thioguanine, 4-thiothymine, 4-thiouracil, O⁶-methylguanine, N⁶-methyladenine, O⁴-methylthymine, 5,6-dihydrothymine, 5,6-dihydrouracil, pyrazolo[3,4-D]pyrimidines (see, e.g., U.S. Pat. Nos. 6,143,877 and 6,127,121 and PCT published application WO 01/38584), ethenoadenine, indoles such as nitroindole and 4-methylindole, and pyrroles such as nitropyrrole. Certain exemplary nucleotide bases can be found, e.g., in Fasman, 1989, Practical Handbook of Biochemistry and Molecular Biology, pp. 385-394, CRC Press, Boca Raton, Fla., and the references cited therein.

"Nucleotide" refers to a phosphate ester of a nucleoside, as a monomer unit or within a nucleic acid. "Nucleotide 5'-triphosphate" refers to a nucleotide with a triphosphate ester group at the 5' position, and are sometimes denoted as "NTP", or "dNTP" and "ddNTP" to particularly point out the structural features of the ribose sugar. The triphosphate ester group can include sulfur substitutions for the various oxygens, e.g. α-thio-nucleotide 5'-triphosphates. For a review of nucleic acid chemistry, see: Shabarova, Z. and Bogdanov, A. Advanced Organic Chemistry of Nucleic Acids, VCH, New York, 1994. The term nucleotide also encompasses nucleotide analogs. The sugar can be substituted or unsubstituted. Exemplary riboses include, but are not limited to, 2'-(C1-C6)alkoxyribose, 2'-(CS-C14)aryloxyribose, 2',3'-didehydroribose, 2'-deoxy-3'-haloribose, 2'-deoxy-3'-fluororibose, 2'-deoxy-3'-chlororibose, 2'-deoxy-3'-aminoribose, 2'-deoxy-3'-(C1-C6)alkylribose, 2'-deoxy-3'-(C1-C6)alkoxyribose and 2'-deoxy-3'-(C5-C14)aryloxyribose, ribose, 2'-deoxyribose, 2',3'-dideoxyribose, 2'-haloribose, 2'-fluororibose, 2'-chlororibose, and 2'-alkylribose, e.g., 2'-O-methyl, 4'-α-anomeric nucleotides, 1'-α-anomeric nucleotides, 2'-4'- and 3'-4'-linked and other "locked" or "LNA", bicyclic sugar modifications (see, e.g., PCT published application nos. WO 98/22489, WO 98/39352; and WO 99/14226). Exemplary LNA sugar analogs within a polynucleotide include, but are not limited to, the structures: 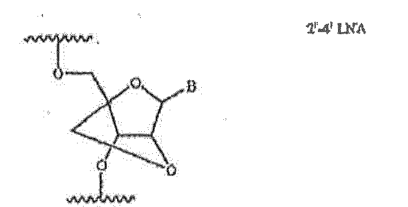 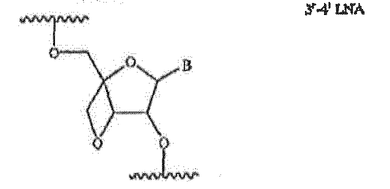 where B is any nucleotide base.

LNAs are a class of nucleic acid analogues that can form base-pairs according to standard Watson-Crick base pairing rules. Oligonucleotides incorporating LNA have increased thermal stability and improved discriminative power with respect to their nucleic acid targets. LNAs for oligonucleotide synthesis are commercially available from various companies such as, for example, Exiqon™ in Denmark (see, world wide web:exiqon.com and U.S. Pat. No. 6,670,461).

The term "nucleotide analog," as used herein, refers to any non-adenine, non-thymine, non-guanine, non-cytosine, non-uracil nucleic acid (where each of "adenine," "thymine," "guanine," "cytosine" and "uracil" only refers to the naturally occurring nucleic acid). As will be appreciated by one of skill in the art, the nucleotide analog will still base pair with one of the above. Nucleotide analogs encompass high stability nucleotides and therefore at least encompass PNA (peptide nucleic acids), LNA (looked nucleic acids), a 2'-O-Methyl nucleic acid, a 2'-O-Alkyl nucleic acid, a 2'-fluoro nucleic acid, a nucleic acid including a phosphorothioate linkage, or any combination thereof. In some embodiments, nucleotide analog refers to embodiments in which the pentose sugar and/or the nucleotide base and/or one or more of the phosphate esters of a nucleotide can be replaced with its respective analog. In, some embodiments, exemplary pentose sugar analogs are those described above. In some embodiments, the nucleotide analogs have a nucleotide base analog as described above. In some embodiments, exemplary phosphate ester analogs include, but are not limited to, alkylphosphonates, methylphosphonates, phosphoramidates, phosphotriesters, phosphorothioates, phosphorodithioates, phosphoroselenoates, phosphorodiselenoates, phosphoroanilothioates, phosphoroanilidates, phosphoroamidates, boronophosphates, etc., and can include associated counterions. Also included within the definition of "nucleotide analog" are nucleotide analog monomers that can be polymerized into polynucleotide analogs in which the DNA/RNA phosphate ester and/or sugar phosphate ester backbone is replaced with a different type of internucleotide linkage. Exemplary polynucleotide analogs include, but are not limited to, peptide nucleic acids, in which the sugar phosphate backbone of the polynucleotide is replaced by a peptide backbone. Exemplary modified pentose portions include but are not limited to 2'- or 3'-modifications where the 2'- or 3'-position is hydrogen, hydroxy, alkoxy, e.g., methoxy, ethoxy, allyloxy, isopropoxy, butoxy, isobutoxy and phenoxy, azido, amino or alkylamino, fluoro, chloro, bromo and the like. Modified internucleotide linkages include phosphate analogs, analogs having achiral and uncharged intersubunit linkages (e.g., Sterchak, E. P., et al., Organic Chem, 52:4202 (1987)), and uncharged morpholino-based polymers having achiral intersubunit linkages (e.g., U.S. Pat. No. 5,034,506). Another exemplary class of polynucleotide analogs where a conventional sugar and internucleotide linkage has been replaced with a 2-aminoethylglycine amide backbone polymer is peptide nucleic acid (PNA) (e.g., Nielsen et al., Science, 254:1497-1500 (1991); Egholm et al., J. Am. Chem. Soc., 114: 1895-197 (1992)).

As used herein, the terms "polynucleotide," "oligonucleotide," and "nucleic acid" are used interchangeably and mean single-stranded and double-stranded polymers of nucleotide monomers, including 2'-deoxyribonucleotides (DNA) and ribonucleotides (RNA) linked by internucleotide phosphodiester bond linkages, or internucleotide analogs, and associated counter ions, e.g., H⁺, NH₄⁺, trialkylammonium, Mg²⁺, Na⁺ and the like. A nucleic acid can be composed entirely of deoxyribonucleotides, entirely of ribonucleotides, or chimeric mixtures thereof. The nucleotide monomer units can include any of the nucleotides described herein, including, but not limited to, naturally occurring nucleotides and nucleotide analogs. Nucleic acids typically range in size from a few monomeric units, e.g. 5-40 when they are sometimes referred to in the art as oligonucleotides, to several thousands of monomeric nucleotide units. Unless denoted otherwise, whenever a nucleic acid sequence is represented, it will be understood that the nucleotides are in 5' to 3' order from left to right and that "A" denotes deoxyadenosine or an analog thereof, "C" denotes deoxycytidine or an analog thereof, "G" denotes deoxyguanosine or an analog thereof, "T" denotes thymidine or an analog thereof, and "U" denotes uridine or an analog thereof, unless otherwise noted.

Nucleic acids also include, but are not limited to, genomic DNA, cDNA, hnRNA, mRNA, rRNA, tRNA, fragmented nucleic acid, nucleic acid obtained from subcellular organelles such as mitochondria or chloroplasts, and nucleic acid obtained from microorganisms or DNA or RNA viruses that can be present on or in a biological sample. Nucleic acids include, but are not limited to, synthetic or in vitro transcription products.

The terms "nucleic acid," "polynucleotide," and "oligonucleotide" can also include nucleic acid analogs, polynucleotide analogs, and oligonucleotide analogs. The terms "nucleic acid analog", "polynucleotide analog" and "oligonucleotide analog" are used interchangeably and, as used herein, refer to a nucleic acid that contains at least one nucleotide analog and/or at least one phosphate ester analog and/or at least one pentose sugar analog. Also included within the definition of nucleic acid analogs are nucleic acids in which the phosphate ester and/or sugar phosphate ester linkages are replaced with other types of linkages, such as N-(2-aminoethyl)-glycine amides and other amides (see, e.g., Nielsen et al., 1991, Science 254:1497-1500; WO 92/20702; U.S. Pat. No. 5,719,262; U.S. Pat. No. 5,698,685;); morpholinos (see, e.g., U.S. Pat. No. 5,698,685; U.S. Pat. No. 5,378,841; U.S. Pat. No. 5,185,144); carbamates (see, e.g., Stirchak & Summerton, 1987, J. Org. Chem. 52: 4202); methylene(methylimino) (see, e.g., Vasseur et al., 1992, J. Am. Chem. Soc. 114:4006); 3'-thioformacetals (see, e.g., Jones et al., 1993, J. Org. Chem. 58: 2983); sulfamates (see, e.g., U.S. Pat. No. 5,470,967); 2-aminoethylglycine, commonly referred to as PNA (see, e.g., Buchardt, WO 92/20702; Nielsen (1991) Science 254:1497-1500); and others (see, e.g., U.S. Pat. No. 5,817,781; Frier & Altman, 1997, Nucl. Acids Res. 25:4429 and the references cited therein). Phosphate ester analogs include, but are not limited to, (i) C₁-C₄ alkylphosphonate, e.g. methylphosphonate; (ii) phosphoramidate; (iii) C₁-C₆ alkyl-phosphotriester; (iv) phosphorothioate; and (v) phosphorodithioate.

An "STR locus" refers to a region of a chromosome containing repeated units that vary in number among certain individuals of a given species, such as humans. The repeats are not necessarily perfect repeats and may contain interruptions. The term "STR locus" encompasses a copy of such a chromosomal region produced, for example, by an amplification reaction. Examples of STR loci can include, but are not limited to, TH01, TPOX, CSF1PO, vWA, FGA, D3S1358, D5S818, D7S820, D13S317, D16S539, D8S1179, D18S51, D21S11, D2S1338, D3S1539, D4S2368, D9S930, D10S1239, D14S118, D14S548, D14S562, D16S490, D16S753, D17S1298, D17S1299, D19S253, D19S433, D20S481, D22S683, HUMCSF1PO, HUMTPOX, HUMTH01, HUMF13AO1, HUMBFXIII, HUMLIPOL, HUMvWFA31. Examples of multiplex PCR of STR markers can be found in U.S. Pat. Nos. 7008771, 6767703, 6479235, 6221598.

As will be appreciated by one of skill in the art, a primer that is configured to amplify or allow amplification of a sequence from one of the above loci can have a sequence that hybridizes within the loci or on either side of the loci.

The term "CODIS loci" as used herein refers to the STR loci designated by the FBI's "Combined DNA Index System." Thirteen core STR loci are TH01, TPOX, CSF1PO, vWA, FGA, D3S1358, D5S818, D7S820, D13S317, D16S539, D8S1179, D18S51, and D21S11. (See, e.g., Butler, Forensic DNA Typing, Academic Press (2001), at page 63.) The FBI may add additional loci to the listed set of 13 loci.

The term "amplification product" refers to the product of an amplification reaction including, but not limited to, primer extension, the polymerase chain reaction, RNA transcription, and the like. Thus, exemplary amplification products can comprise one or more products selected from primer extension products, PCR amplicons, RNA transcription products, and the like.

As used herein, the term "amplifying" refers to any means by which at least a part of a target polynucleotide, target polynucleotide surrogate, or combinations thereof, is reproduced, typically in a template-dependent manner, including without limitation, a broad range of techniques for amplifying nucleic acid sequences, either linearly or exponentially. Exemplary means for performing an amplifying step include ligase chain reaction (LCR), ligase detection reaction (LDR), ligation followed by Q-replicase amplification, PCR, primer extension, strand displacement amplification (SDA), hyperbranched strand displacement amplification, multiple displacement amplification (MDA), nucleic acid strand-based amplification (NASBA), two-step multiplexed amplifications, rolling circle amplification (RCA) and the like, including multiplex versions or combinations thereof, for example but not limited to, OLA/PCR, PCR/OLA, LDR/PCR, PCR/PCR/LDR, PCR/LDR, LCR/PCR, PCR/LCR (also known as combined chain reaction--CCR), and the like. Descriptions of such techniques can be found in, among other places, Sambrook et al. Molecular Cloning, 3rd Edition; Ausbel et al.; PCR Primer: A Laboratory Manual, Diffenbach, Ed., Cold Spring Harbor Press (1995); The Electronic Protocol Book, Chang Bioscience (2002), Msuih et al., J. Clin. Micro. 34:501-07 (1996); The Nucleic Acid Protocols Handbook, R. Rapley, ed., Humana Press, Totowa, N.J. (2002); Abramson et al., Curr Opin Biotechnol. 1993 February; 4(1):41-7, U.S. Pat. No. 6,027,998; U.S. Pat. No. 6,605,451, Barany et al., PCT Publication No. WO 97/31256; Wenz et al., PCT Publication No. WO 01/92579; Day et al., Genomics, 29(1): 152-162 (1995), Ehrlich et al., Science 252:1643-50 (1991); Innis et al., PCR Protocols: A Guide to Methods and Applications, Academic Press (1990); Favis et al., Nature Biotechnology 18:561-64 (2000); and Rabenau et al., Infection 28:97-102 (2000); LCR Kit Instruction Manual, Cat. #200520, Rev. #050002, Stratagene, 2002; Barany, Proc. Natl. Acad. Sci. USA 88:188-93 (1991); Bi and Sambrook, Nucl. Acids Res. 25:2924-2951 (1997); Zirvi et al., Nucl. Acid Res. 27:e40i-viii (1999); Dean et al., Proc Natl Acad Sci USA 99:5261-66 (2002); Barany and Gelfand, Gene 109:1-11 (1991); Walker et al., Nucl. Acid Res. 20:1691-96 (1992); Polstra et al., BMC Inf Dis. 2:18-(2002); Lage et al., Genome Res. 2003 February; 13(2):294-307, and Landegren et al., Science 241:1077-80 (1988), Demidov, V., Expert Rev Mol Diagn. 2002 November;2(6):542-8., Cook et al., J Microbiol Methods. 2003 May;53(2):165-74, Schweitzer et al., Curr Opin Biotechnol. 2001 February; 12(1):21-7, U.S. Pat. No. 5,830,711, U.S. Pat. No. 6,027,889, U.S. Pat. No. 5,686,243, Published P.C.T. Application WO0056927A3, and Published P.C.T. Application WO9803673A1. In some embodiments, newly-formed nucleic acid duplexes are not initially denatured, but are used in their double-stranded form in one or more subsequent steps. In some embodiments of the present teachings, unconventional nucleotide bases can be introduced into the amplification reaction products and the products treated by enzymatic (e.g., glycosylases) and/or physical-chemical means in order to render the product incapable of acting as a template for subsequent amplifications. In some embodiments, uracil can be included as a nucleobase in the reaction mixture, thereby allowing for subsequent reactions to decontaminate carryover of previous uracil-containing products by the use of uracil-N-glycosylase (see for example Published P.C.T. Application WO9201814A2, U.S. Pat. No. 5,536,649, and U.S. Provisional Application 60/584,682 to Andersen et al., wherein UNG decontamination and phosphorylation are performed in the same reaction mixture, which further comprises a heat-activatable ligase.). In some embodiments of the present teachings, any of a variety of techniques can be employed prior to amplification in order to facilitate amplification success, as described for example in Radstrom et al., Mol Biotechnol. 2004 February; 26(2):133-46. In some embodiments, amplification can be achieved in a self-contained integrated approach comprising sample preparation and detection, as described for example in U.S. Pat. Nos. 6,153,425 and 6,649,378. Reversibly modified enzymes, for example but not limited to those described in U.S. Pat. No. 5,773,258, are also within the scope of the disclosed teachings. Those in the art will understand that any protein with the desired enzymatic activity can be used in the disclosed methods and kits. Descriptions of DNA polymerases, including reverse transcriptases, uracil N-glycosylase, and the like, can be found in, among other places, Twyman, Advanced Molecular Biology, BIOS Scientific Publishers, 1999; Enzyme Resource Guide, rev. 092298, Promega, 1998; Sambrook and Russell; Sambrook et al.; Lehninger; PCR: The Basics; and Ausbel et al.

A "primer nucleic acid" or "primer" refers to a nucleic acid that can hybridize to a target or template nucleic acid and permit chain extension or elongation using, e.g., a nucleotide incorporating biocatalyst, such as a polymerase under appropriate reaction conditions. Such conditions can include the presence of one or more deoxyribonucleoside triphosphates and the nucleotide incorporating biocatalyst, in a suitable buffer ("buffer" includes substituents which are cofactors, or which affect pH, ionic strength, etc.), and at a suitable temperature. A primer nucleic acid can be, for example, a natural or synthetic oligonucleotide (e.g., a single-stranded oligodeoxyribonucleotide, etc.).

The term "set of primers" refers to at least one primer that, under suitable conditions, specifically hybridizes to and amplifies a target sequence. In some embodiments, a set of primers comprises at least two primers.

The term "STR-specific primer set" refers to at least two primers that are used for analyzing a STR locus.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

As used herein, the term "mobility modifier" refers to a polymer chain that imparts to an oligonucleotide an electrophoretic mobility in a sieving or non-sieving matrix that is distinctive relative to the electrophoretic mobilities of the other polymer chains in a mixture. Typically, a mobility modifier changes the charge/translational frictional drag when hybridized or bound to the element; or imparts a distinctive mobility, for example but not limited to, a distinctive elution characteristic in a chromatographic separation medium or a distinctive electrophoretic mobility in a sieving matrix or non-sieving matrix, when hybridized or bound to the corresponding element; or both (see, e.g., U.S. Pat. Nos. 5,470,705 and 5,514,543). For various examples of mobilitity modifiers see for example U.S. Pat. Nos. 6,395,486, 6,358,385, 6,355,709, 5,916,426, 5,807,682, 5,777,096, 5,703,222, 5,556,7292, 5,567,292, 5,552,028, 5,470,705, and Barbier et al., Current Opinion in Biotechnology, 2003, 14:1:51-57. In some embodiments, at least one mobility modifier comprises at least one nucleotide polymer chain, including without limitation, at least one oligonucleotide polymer chain, at least one polynucleotide polymer chain, or both at least one oligonucleotide polymer chain and at least one polynucleotide polymer chain (see for example Published P.C.T. application WO9615271A1, as well as product literature for Keygene SNPWave^{™} for some examples of using known numbers of nucleotides to confer mobility to ligation products). In some embodiments, at least one mobility modifier comprises at least one non-nucleotide polymer chain. Exemplary non-nucleotide polymer chains include, without limitation, peptides, polypeptides, polyethylene oxide (PEO), or the like. In some embodiments, at least one polymer chain comprises at least one substantially uncharged, water-soluble chain, such as a chain composed of PEO units; a polypeptide chain; or combinations thereof. The polymer chain can comprise a homopolymer, a random copolymer, a block copolymer, or combinations thereof. Furthermore, the polymer chain can have a linear architecture, a comb architecture, a branched architecture, a dendritic architecture (*e.g.,* polymers containing polyamidoamine branched polymers, Polysciences, Inc. Warrington, Pa.), or combinations thereof. In some embodiments, at least one polymer chain is hydrophilic, or at least sufficiently hydrophilic when hybridized or bound to an element to ensure that the element-mobility modifier is readily soluble in aqueous medium. Where the mobility-dependent analysis technique is electrophoresis, in some embodiments, the polymer chains are uncharged or have a charge/subunit density that is substantially less than that of its corresponding element. The synthesis of polymer chains useful as mobility modifiers will depend, at least in part, on the nature of the polymer. Methods for preparing suitable polymers generally follow well-known polymer subunit synthesis methods. These methods, which involve coupling of defined-size, multi-subunit polymer units to one another, either directly or through charged or uncharged linking groups, are generally applicable to a wide variety of polymers, such as polyethylene oxide, polyglycolic acid, polylactic acid, polyurethane polymers, polypeptides, oligosaccharides, and nucleotide polymers. Such methods of polymer unit coupling are also suitable for synthesizing selected-length copolymers, e.g., copolymers of polyethylene oxide units alternating with polypropylene units. Polypeptides of selected lengths and amino acid composition, either homopolymer or mixed polymer, can be synthesized by standard solid-phase methods (e.g., Int. J. Peptide Protein Res., 35: 161-214 (1990)). One method for preparing PEO polymer chains having a selected number of hexaethylene oxide (HEO) units, an HEO unit is protected at one end with dimethoxytrityl (DMT), and activated at its other end with methane sulfonate. The activated HEO is then reacted with a second DMT-protected HEO group to form a DMT-protected HEO dimer. This unit-addition is then carried out successively until a desired PEO chain length is achieved (*e.g.,* U.S. Pat. No. 4,914,210; see also, U.S. Pat. No. 5,777,096).

Deoxynucleotide triphosphates ("dNTPs"), which are the building blocks of the amplifying nucleic acid molecules, are typically supplied in standard PCR reactions at a concentration of 40-200 µM each of deoxyadenosine triphosphate ("dATP"), deoxyguanosine triphosphate ("dGTP"), deoxycytidine triphosphate ("dCTP"), and deoxythymidine triphosphate ("dTTP"). Other dNTPs, such as deoxyuridine triphosphate ("dUTP"), and dNTP analogs, and conjugated dNTPs can also be used, and are encompasssed by the term "dNTPs" as used herein. While use of dNTPs at such concentrations is amenable to the methods of the invention, concentrations of dNTPs higher than 200 µM can be advantageous. Thus, in some embodiments of the methods of the invention, the concentration of each dNTP is generally at least 500 µM and can range up to 2 mM. In some further embodiments, concentration of each dNTP can range from 0.5 mM to 1 mM.

As used herein, the term "providing" refers broadly to supplying, obtaining, or possessing something, (*e.g.,* a sample), or making something available, and is not limited in any way by the source or supplier of the thing being provided.

As used herein, "sample" refers to any substance that comprises or is presumed to comprise a nucleic acid of interest (a target nucleic acid sequence) or which is itself a nucleic acid containing or presumed to comprise a target nucleic acid sequence of interest. The term "sample" thus includes a sample of nucleic acid (genomic DNA, cDNA, RNA), cell, organism, tissue, fluid, or substance including but not limited to, for example, plasma, serum, spinal fluid, lymph fluid, synovial fluid, urine, tears, stool, external secretions of the skin, respiratory, intestinal and genitourinary tracts, saliva, blood cells, tumors, organs, tissue, samples of in vitro cell culture constituents, natural isolates (such as drinking water, seawater, solid materials), microbial specimens, and objects or specimens that have been "marked" with nucleic acid tracer molecules. The term sample can encompass the actual sample taken for subsequent testing (such as a soil, blood, or piece of cloth for testing). While the sample can change during various stages of processing (e.g., it is taken from a cloth and transferred to a solution in a sterile container) the sample will continuously comprise the target nucleic acid sequence.

The term "location" denotes the area from which the sample was positioned at some point in time. For example, a sample can be located on a blunt instrument, transferred to a piece of cloth, transferred to the soil, and then transferred to a sterile tube. Each of these would be a location of the sample. One of skill in the art will appreciate that many location, and indeed most non-laboratory related locations will have a high likelihood of including contaminants, such as PCR inhibitors.

The term "PCR inhibitor" denotes that the presence of the compound reduces the efficiency or effectiveness of a PCR amplification or nucleic acid amplification in general. As will be appreciated by one of skill in the art, a PCR inhibitor does not require that the compound completely inhibit all amplification (such a compound will be denoted as a "complete inhibitor"). Rather, any amount of inhibition can be sufficient, such as 0-1, 1-10, 10-20, 20-30, 30-40, 40-50, 50-60, 60-70, 70-80, 80-90, 90-95, 95-98, 98-99, or 99-100% inhibition. In some embodiments, samples collected from non-laboratory conditions (or locations) are presumed to include a PCR inhibitor. In some embodiments, a sample can be tested to determine if a PCR inhibitor is present (e.g., by running a control PCR). In some embodiments, a PCR inhibitor is presumed when a location of the sample suggests that a PCR inhibitor is likely present. In some embodiments, a PCR inhibitor is selected from at least one of the following: humic acid, bile salt, other salt, complex polysaccharides, collagen, heme, melanin, eumelanin, myoglobin, polysaccharides, proteinases, calcium ions, urea, hemoglobin, lactoferrin, immunoglobulin G, indigo dye, hemoglobin, fulvic acid, divalent cations, chelating molecules, enzymes, and proteinshumic acid, complex polysaccharides, EDTA, EGTA, DNAse, and collagen.

The term "standard stability primer" denotes that the primer is a standard nucleic acid sequence primer. The primer can comprise nucleic acids such as A, T, G, C, or U. The term is generally used herein in comparison to a comparable high stability primer.

The term "comparable" when used in reference to a standard stability primer and a high stability primer denotes that the sequences are identical apart from the noted difference(s) (*e.g.,* the high stability primer will contain one or more nucleic acid analog(s), which will be a comparable replacement to the nucleic acid in the standard stability primer). A nucleic acid replacement is "comparable" when the first and second nucleic acids have the same or similar base pairing selectivity properties (*e.g.,* they both base pair to A over T, G, and, C or they both base pair to C over A, T, or G).

The term "high stability primer" denotes that the primer comprises at least one high stability nucleic acid analog. As will be appreciated by one of skill in the art, in some embodiments, the high stability primer can anneal so as to allow amplification of a STR, such as one of the CODIS sequences.

The term "high stability nucleic acid analog" denotes that the nucleic acid is a nucleic acid analog and that the nucleic acid associates more strongly when base paired to a first natural nucleic acid, than a comparable second natural nucleic acid would bind to the same first nucleic acid, under the same environmental conditions. In some embodiments, this means that the high stability nucleic acid analog has a higher Tm compared to the natural nucleic acid, under the same environmental conditions (*e.g*., PCR conditions). In some embodiments, the high stability nucleic acid analog can be PNA, LNA, a 2'-O-ethyl nucleic acid, a 2'-O-Alkyl nucleic acid, a 2'-fluoro nucleic acid, a nucleic acid including a phosphorothioate linkage, or any combination thereof. A high stability nucleic acid analog is "comparable" to a second natural nucleic acid when they both have the same or similar base pairing selectivity properties (*e.g.,* they both base pair to A over T, G, and, C or they both base pair to C over A, T, or G). The relative strength of the base pairing interactions can be determined in a number of ways, for example, computational modeling, standard knowledge of one of skill in the art, or through a melting point analysis of the base paired molecules. In some embodiments, this can be examined by preparing two primers, a first primer having the possible high stability nucleic acid analog (*e.g*., ATC(LNA G)GC) and a comparable standard stability primer (*e.g.,* ATCGGC) and comparing the melting point of the two primer to the same complementary sequence (*e.g.,* TAGCCG).

As used herein, "target nucleic acid sequence" refers to a region of a nucleic acid that is to be either replicated, amplified, and/or detected. In some embodiments, the "target nucleic acid sequence" or "template nucleic acid sequence" resides between two primer sequences used for amplification. As will be appreciated by one of skill in the art, the target nucleic acid can be from an individual or group that is to be identified or matched via the characterization of the target nucleic acid sequence. Such an individual can be denoted as the "target individual." In some embodiments, the target individual is known. In some embodiments, the target individual is not known.

In this application, a statement that one sequence is the same as or is complementary to another sequence encompasses situations where both of the sequences are completely the same or complementary to one another, and situations where only a portion of one of the sequences is the same as, or is complementary to, a portion or the entire other sequence. In this situation, the term "sequence" encompasses, but is not limited to, nucleic acid sequences, polynucleotides, oligonucleotides, probes, primers, primer-specific portions, and target-specific portions.

In this application, a statement that one sequence is complementary to another sequence encompasses situations in which the two sequences have mismatches. In this situation, the term "sequence" encompasses, but is not limited to, nucleic acid sequences, polynucleotides, oligonucleotides, probes, primers, primer-specific portions, and target-specific portions. Despite the mismatches, the two sequences should selectively hybridize to one another under appropriate conditions.

In this application, a statement that one sequence hybridizes or binds to another sequence encompasses embodiments where the entirety of both of the sequences hybridize or bind to one another, and embodiments where only a portion of one or both of the sequences hybridizes or binds to the entire other sequence or to a portion of the other sequence.

### Exemplary Embodiments

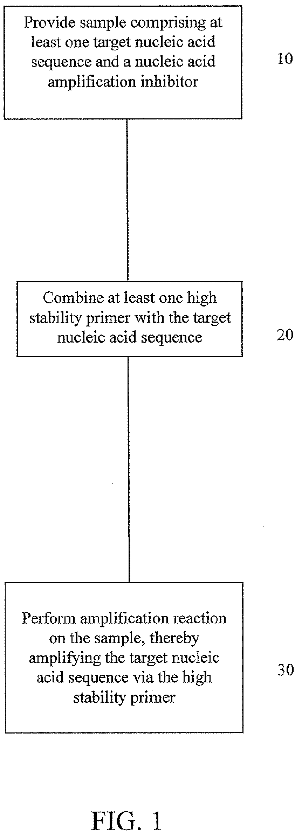

**FIG. 1** is a flow chart of one embodiment of a method for amplifying a target nucleic acid sequence in a sample comprising (or suspected of comprising) at least one nucleic acid amplification inhibitor. In some embodiments, this involves providing a sample comprising at least one target nucleic acid sequence and a nucleic acid amplification inhibitor 10. Next, at least one high stability primer is combined with the target nucleic acid sequence 20. The high stability primer can comprise at least one high stability nucleic acid analog. Following this, an amplification reaction is performed on the sample 30. As will be appreciated by one of skill in the art, one need not add or know for certain that the amplification inhibitor is present in the sample.

As noted above, the sample can be any substance containing or presumed to contain a nucleic acid of interest (a target nucleic acid sequence) or which is itself a nucleic acid containing or presumed to contain a target nucleic acid sequence of interest. In some embodiments, the sample comprises a nucleic acid (genomic DNA, cDNA, RNA), a cell, an organism, a tissue, a fluid, or a substance including but not limited to, for example, plasma, serum, spinal fluid, lymph fluid, synovial fluid, urine, tears, stool, external secretions of the skin, respiratory, intestinal and genitourinary tracts, saliva, blood cells, tumors, organs, tissue, samples of in vitro cell culture constituents, natural isolates (such as drinking water, seawater, solid materials), microbial specimens, objects or specimens that have been "marked" with nucleic acid tracer molecules, or any combination thereof.

The sample comprising the target nucleic acid sequence can comprise biological material from any source. The sample can be provided from any of a wide variety of sources, and need not be directly provided from the original biological source of the nucleic acid. In some embodiments, the sample can be from a location that was believed to, or would be assumed to, be contaminated with a composition that can inhibit nucleic acid amplification. In some embodiments, a sample comprising a target nucleic acid sequence can be biological material obtained, e.g., from a crime scene or from a site containing human or animal remains, such as an archeological site or a disaster site. In some embodiments, nucleic acid is extracted from the sample. See, e.g., Butler, Forensic DNA Typing, at pages 28-32. In some embodiments, the sample, including the nucleic acid, can be degraded or present in low amounts. In some embodiments, the sample can comprise at least a target nucleic acid sequence from an individual.

In some embodiments, the location of the sample can be (or at one point could have been) an indoor environment. The indoor environment can be, for example, inside a residential dwelling, a house, an apartment, a condominium, a hotel, a motel, a government office, a grocery store, a convenience store, an office, an office building, a hospital, a clinic, a church, a restaurant, a shopping mall, a school, a college, a university, a dormitory, a prison, a jail, a garage, or a library. In some embodiments, the sample can be from inside a vehicle, such as a car, an airplane, a train, a bus, a van, an ambulance, a police car, a fire engine, or a taxi. In other embodiments, the sample can be from an outdoors environment. The outdoors environment can be, for example, a park, a yard, a forest, a wood, a street, a highway, schoolyard, a university campus, an office complex grounds, a campground, a jogging path, a hiking trail, a plaza, or a parking lot. In some embodiments, the sample can be from a body of water such as a lake, a pond, an ocean, a river, a creek, a swamp, a pool, or a hot tub. As will be appreciated by one of skill in the art, the sample can be in direct contact with various surfaces of any of the above locations, as well as others.

In some embodiments, the sample can comprise at least a portion of clothing such as jeans, pants, a sweater, a shirt, underwear, a skirt, a dress, a scarf, sneakers, shoes, boots, a uniform, gloves, mittens, socks, stockings, a jacket, or a coat. In some embodiments, the sample can comprise at least a portion of an accessory, such as eyeglasses, jewelry, a handbag, a wig or a purse. In some embodiments, the sample can comprise at least a portion of furniture. The furniture can be, for example, a table, a chair, a car seat, a bed, a crib, a headboard, a stool, a counter, a kitchen appliance, or a lamp. In some embodiments, the sample can comprise fabric. The fabric can comprise, for example, denim, canvas, silk, cotton, rayon, wool, fur, leather, suede, plastic or synthetic fabric. In some embodiments, the sample can comprise paper, furniture, wood, bamboo, plastic, metal, glass, ceramic, plaster, or paint. In some embodiments, the sample can comprise at least portion of upholstery, shower curtain, window curtain, a shade, a blind, a rug, a carpet, a bed sheet, a pillowcase, a bedspread, or a blanket. As will be appreciated by one of skill in the art, the above substances that the sample can comprise can also be characterized as locations upon which the target nucleic acid sequence or sample spends some time. As will be appreciated by one of skill in the art, the sample or target nucleic acid sequence can be directly in contact with the above substances.

In some embodiments, the sample comprises (or is presumed to comprise) a nucleic acid and a nucleic acid amplification inhibitor. The nucleic acid comprises a target nucleic acid sequence. In some embodiments, the target nucleic acid sequence can comprise a nucleic acid, a nucleic acid analog, a polynucleotide analogs, and oligonucleotide analogs. In some embodiments, the target nucleic acid sequence can comprise naturally occurring DNA. In some embodiments, the target nucleic acid sequence can comprise at least one short tandem repeat (STR).

A target nucleic acid sequence for use with the present invention can be derived from any living, or once living, organism, including but not limited to prokaryote, eukaryote, plant, animal, and virus. The target nucleic acid sequence can originate from a nucleus of a cell, e.g., genomic DNA, or can be extranuclear nucleic acid, e.g., plasmid, mitochondrial nucleic acid, various RNAs, and the like. The target nucleic acid sequence can be first reverse-transcribed into cDNA if the target nucleic acid is RNA. Furthermore, the target nucleic acid sequence can be present in a double stranded or single stranded form.

The nucleic acid amplification inhibitor can be, for example, a PCR inhibitor or a compound or material that is capable of damaging nucleic acids. Examples comprise, but are not limited to, humic acid, bile salt, other salt, complex polysaccharides, collagen, heme, melanin, eumelanin, myoglobin, polysaccharides, proteinases, calcium ions, urea, hemoglobin, lactoferrin, immunoglobulin G, indigo dye, hemoglobin, fulvic acid, divalent cations, chelating molecules, enzymes, proteins, complex polysaccharides, EDTA, EGTA, DNAse, and collagen.

In some embodiments, the nucleic acid amplification inhibitor can be a contaminant in the sample. In some embodiments, the nucleic acid amplification inhibitor can be environmental. One or more nucleic acid amplification inhibitors can be present in the sample. In some embodiments, the sample comprises at least one nucleic acid amplification inhibitor. In some embodiments, the sample comprises at least two, three, four, five, six, seven, eight, nine, ten, eleven, twelve, thirteen, fourteen, fifteen, sixteen, seventeen, eighteen, nineteen, or twenty nucleic acid amplification inhibitors.

In some embodiments, the nucleic acid amplification inhibitor can be a substance that may not always act as an inhibitor but is capable of inhibiting nucleic acid amplification in some situations, for example, at some concentrations. For example, a salt such as MgCl₂ may not inhibit nucleic acid amplification at some concentrations, but at higher concentrations, it can act as an inhibitor. As will be appreciated by one of skill in the art, in such situations, the substance will only be deemed a "nucleic acid inhibitor" if it is present, under conditions of the actual nucleic acid amplification, at a level sufficient to at least partially inhibit amplification. A method for determining the presence of a nucleic acid amplification inhibitor in a sample is provided in the Examples section below. In some embodiments, the nucleic acid inhibitor can be identified in a sample. In other embodiments, the nucleic acid inhibitor need not be identified in the sample. In some embodiments, the nucleic acid inhibitor only functions as an inhibitor or has no function at all (thus excluding compositions such as MgCl₂, discussed above). In some embodiments, the nucleic acid amplification inhibitor is an environmental condition, such as temperature. In some embodiments, conditions (such as temperature) are excluded as possibilities as nucleic acid amplification inhibitors.

In some embodiments, one practicing some of the presently disclosed techniques makes a decision that one of the locations that a sample was in suggests a likelihood of an amplification inhibitor. After making this decision, they then apply the remainder of the disclosed method, involving a high stability primer.

In some embodiments, the methods can comprise at least one high stability primer. In other embodiments, the methods can comprise at least two high stability primers. In some embodiments, the methods can comprise at least three, four, five, six, seven, eight, nine, ten, eleven, twelve, thirteen, fourteen, fifteen, sixteen, seventeen, eighteen, nineteen, twenty or more high stability primers, each high stability primer having a different sequence and amplifying a different loci.

In some embodiments, the high stability primer comprises at least one high stability nucleic acid analog. In some embodiments, the high stability primer comprises at least two, three, four, five, six, seven, eight, nine, ten, eleven, twelve, thirteen, fourteen, fifteen, sixteen, seventeen, eighteen, nineteen, twenty or more high stability nucleic acid analogs. In some embodiments, the high stability nucleic acid analog is PNA, LNA, a 2'-O-Methyl nucleic acid, a 2'-O-Alkyl nucleic acid, a 2'-fluoro nucleic acid, a nucleic acid including a phosphorothioate linkage, or any combination thereof. In some embodiments, at least 1% of nucleic acids in the high stability primer are nucleic acid analogs, *e.g.,* 1-5, 5-10, 10-15, 15-20, 20-25, 25-30, 30-35, 35-40, 40-50, 50-60, 60-70, 70-80, 80-90, 90-100% of the nucleic acids in the high stability primer are nucleic acid analogs. In some embodiments, the nucleic acid analog(s) are located at the 5' end of the primer. In some embodiments, the nucleic acid analog(s) are located at the 3' end of the primer. In some embodiments, the nucleic acid analog(s) are located at in the center of the primer. In some embodiments, the nucleic acid analogs are distributed evenly throughout the primer. In some embodiments, the nucleic acid analogs are located adjacent to one another in the sequence. In some embodiments, the nucleic acid analogs are separated by natural nucleic acids. In some embodiments, different types nucleic acid analogs (*e.g.,* LNA and PNA) are used in a single primer. In some embodiments, all of the nucleic acid analogs are the same type, but need not be the same nucleic acid (e.g., A vs. G analog versions). In some embodiments, the various high stability primers employ different nucleic acid analogs. In some embodiments, multiple high stability primers are used in a single reaction, and the multiple high stability primers, while binding to a same target nucleic acid sequence, comprises a different nucleic acid analog. In some embodiments, this overlap in binding ability can compensate for the presence of various PCR inhibitors.

In some embodiments, the high stability primer can have a higher melting point temperature than a second primer (*e.g.,* a standard stability primer) that is almost identical to the high stability primer, except that the second primer consists of natural nucleic acids, thereby lacking a high stability nucleic acid analog. In some embodiments, the high stability primer can have a higher melting point temperature than a second primer that is almost identical to the high stability primer, except that the second primer comprises at least one high stability nucleic acid analog at a different position.

As will be appreciated by one of skill in the art, the above comparison of "high stability primers" and "standard stability primers" frequently characterizes the high stability primer as having at least one nucleic acid "replaced" with a nucleic acid analog. This is for ease of description only and does not require that the primer actually have a nucleic acid physically "replaced" before it is first used in order for it to be called a "high stability primer." That is, how the primer is designed or actually created does not alter whether or not it is a high stability primer. The methods described herein do not require an actual step of replacement of a standard with a nucleic acid analog. In other words, high stability primers can be prepared without any intermediate step involving a standard stability primer. The "replacement" or "substitution" language used herein is simply for convenience and for comparison purposes with standard stability primers.

In some embodiments, the high stability primer further comprises a mobility modifier. Mobility modifiers are known in the art, and are described in more detail above. In some embodiments, the mobility modifier can be polyethylene oxide, polyglycolic acid, polylactic acid, polypeptide, oligosaccharide, polyurethane, polyamide, polysulfonamide, polysulfoxide, polyphosphonate, or block copolymers thereof. As noted above, mobility modifiers allow the mobility of each primer to be arbitrarily defined, regardless of oligonucleotide length or sequence.

The high stability primer can specifically hybridize to the target nucleic acid sequence. In some embodiments, the high stability primer hybridizes to the target nucleic acid sequence in a manner to allow amplification of a short tandem repeat. In some embodiments, the standard stability primer (on which the high stability primer is based or is comparable to) can be from a STR-specific primer set (*e.g.*, a primer or primer set that will bind to or allow amplification of a STR). In some embodiments, the primer can be from a CODIS-specific primer set. In some embodiments, the primer can be an Amelogenin LNA™-containing oligonucleotide. In some embodiments,

A wide variety of nucleic acid sequences can be amplified with the high stability primer. In some embodiments, a nucleic acid sequence from at least one locus can be amplified. In some embodiments, a nucleic acid sequence from at least one STR locus can be amplified. In some embodiments, the amplification can amplify a nucleic acid sequence from a locus such as, for example, Amelogenin, TH01, TPOX, CSF1PO, vWA, FGA, D3S1358, D5S818, D7S820, D13S317, D16S539, D8S1179, D18S51, D21S11, D2S1338, D3S1539, D4S2368, D9S930, D10S1239, D14S118, D14S548, D14S562, D16S490, D16S753, D17S1298, D17S1299, D19S253, D19S433, D20S481, D22S683, HUMCSF1PO, HUMTPOX, HUMTH01, HUMF13A01, HUMBFXIII, HUMLIPOL, HUMvWFA31, or any combination thereof. In some embodiments, the amelogenin sequence is amplified.

In some embodiments, more than one locus is amplified in one reaction. In some embodiments, two, three, four, five, six, seven, eight, nine, ten, eleven, twelve, thirteen, fourteen, fifteen, sixteen, seventeen, eighteen, nineteen, twenty or more loci are co-amplified. In some embodiments employing multiplex co-amplification, not all of the primer pairs comprise a high stability primer.

After amplification, the products from the PCR reactions can be analyzed, resolved, and/or characterized by any of a variety of methods known in the art. For example, PCR reactions can be analyzed by denaturing samples and separating using gel electrophoresis or a capillary electrophoresis protocol. The results from this can then allow one to determine the number of repeats of the STR sequence that are present.

Nucleic acid amplification and hybridization procedures are known in the art. In some embodiments, amplification can be achieved via PCR. For example, PCR amplification protocols are provided in the Applied Biosystems® AmpF/STR® SEfiler PCR Amplification Kit User's Manual.

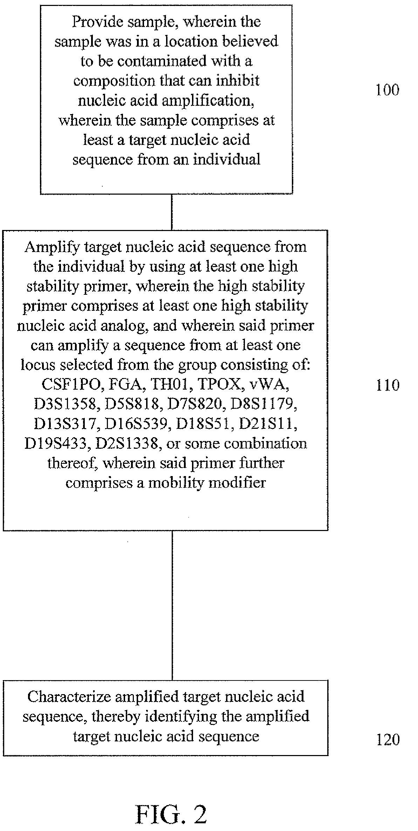

**FIG. 2** is a flow chart of some embodiments of a method for amplifying a target nucleic acid sequence in a sample comprising at least one nucleic acid amplification inhibitor. In some embodiments, this involves providing a sample, wherein the sample was in a location believed to be contaminated with a composition that can inhibit nucleic acid amplification, wherein the sample comprises at least a target nucleic acid sequence from an individual 100.

Next, the target nucleic acid sequence from the individual is amplified using at least one high stability primer, wherein the high stability primer comprises at least one high stability nucleic acid analog, and wherein the primer can amplify a sequence from at least one locus selected from at least one of the following: CSF1PO, PGA, TH01, TPOX, vWA, D3S1358, D5S818, D7S820, D8S1179, D13S317, D16S539, D18S51, D21S11, D19S433, D2S1338, or some combination thereof, wherein the primer further comprises a mobility modifier 110.

Following this, the amplified target nucleic acid sequence is characterized, thereby identifying the amplified target nucleic acid sequence 120. In some embodiments, the characterization is achieved by determining the number of STRs that are present in at least one, and preferably more than one of the above loci for the amplified product.

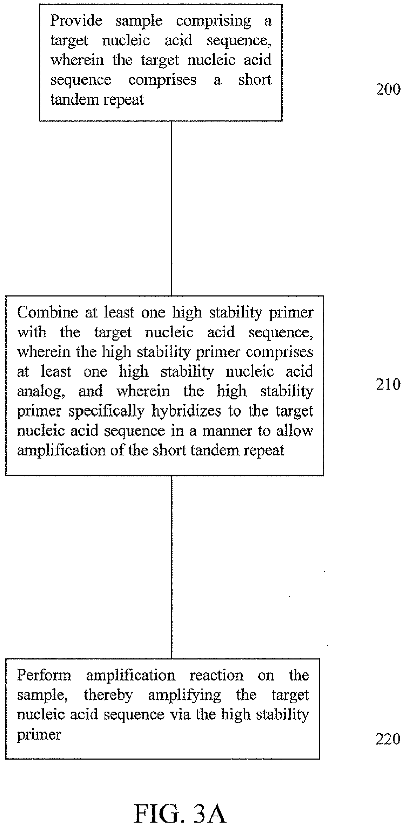

**FIG. 3A** is a flow chart of some embodiments of a method for amplifying a target nucleic acid sequence in a sample comprising at least one nucleic acid amplification inhibitor. In some embodiments, this involves providing a sample comprising a target nucleic acid sequence, wherein the target nucleic acid sequence comprises a short tandem repeat that can be used in the identification of a source (*e.g.,* target individual) of the target nucleic acid sequence 200. Next, at least one high stability primer is combined with the target nucleic acid sequence 210. The high stability primer comprises at least one high stability nucleic acid analog, and specifically hybridizes to the target nucleic acid sequence in a manner to allow amplification of the short tandem repeat. Following this, an amplification reaction is performed on the sample, thereby amplifying the target nucleic acid sequence via the high stability primer 220. In some embodiments, the location of high stability nucleic acid analog is not at the 3' end of the high stability primer. In some embodiments, the high stability nucleic acid analog is not the last nucleic acid at the 3' end of the high stability primer.

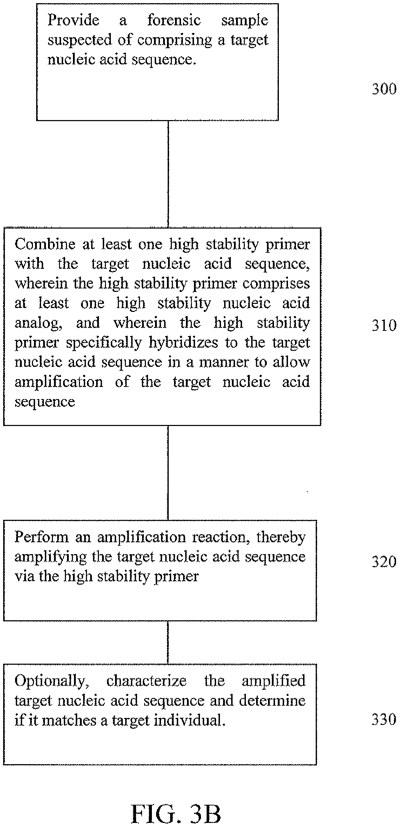

In some embodiments, a PCR inhibitor is not required to be present or even suspected of being present. In some embodiments, it can be useful to use the high stability primers whenever a forensic sample is to be amplified. One such embodiment is depicted in **FIG. 3B****.** One can first provide a forensic sample that is suspected of comprising a target nucleic acid sequence **300.** The forensic sample need not be collected by the person performing the present technique. In some embodiments, the sample is cleaned to some extent. In some embodiments, the forensic sample is treated so as to place the target nucleic acid in a solution or buffer for subsequent amplification. In some embodiments, the sample is treated so as to release the target nucleic acid from cells or cellular components that can be present in the sample. As will be appreciated by one of skill in the art, while some collection and/or purification can be desirable, the sample need not be 100% free of contaminants or of PCR inhibitors. In some embodiments, PCR inhibitors are left mixed with the target nucleic acid sequence.

Following this, one can then combine at least one high stability primer with the target nucleic acid sequence, wherein the high stability primer specifically hybridizes to the target nucleic acid sequence in a manner to allow amplification of the target nucleic acid sequence, as shown in step **310.** Following this, one can perform an amplification step to amplify the target nucleic acid sequence via the high stability primer, as shown in step **320.** As will be appreciated by one of skill in the art, there are a variety of amplification techniques that can be used, such as PCR, quantitative PCR, or TAQMAN® PCR. Finally, and optionally, one can characterize the amplified target nucleic acid sequence and then compare that characterization to an individual's (such as a suspect) profile, as in step **330.** In embodiments in which short tandem repeats (STRs) are being amplified and characterized, the various characteristics of the STRs in the sample can be compared to the individual's various STRs to determine if there is a match. As will be appreciated by one of skill in the art, in some embodiments, the other options described herein in regard to the other embodiments can be applied to this method as well.

As will be appreciated by one of skill in the art, the forensic sample can comprise a number of various substances, such as saliva, blood, vaginal fluid, semen, plasma, serum, spinal fluid, lymph fluid, synovial fluid, urine, tears, and stool. In some embodiments, forensic sample comprises an external secretion from an organ selected from the group consisting of the skin, mouth, lung, nose, eye, ear, navel, intestinal tract, genitourinary tract, and any combination thereof. In some embodiments, the sample includes a target nucleic acid sequence from an animal. In some embodiments, the animal is a human.

In some embodiments, a forensic sample is one that can be used to address a question of interest to the legal system; however, it need not be limited to this in all embodiments. For example, in some embodiments, a forensic sample is one that someone desires to identify or characterize. The identification or characterization can be with regard to a known or unknown source (*e.g*., a candidate target individual). In some embodiments, a forensic sample is one that one desires to identify a source of.

In some embodiments, STRs can be included in the target nucleic acid sequence; however, it is not required that STRs be present in all embodiments. For example, in some embodiments, any nucleic acid sequence that can be useful in forensic analysis can be a target nucleic acid sequence. In some embodiments, the target nucleic acid sequence allows one to determine a source of a substance (such as a tissue sample, blood, etc) found at one location with an individual. In some embodiments, it allows one to rule out an individual as a possible source of the substance.

In some embodiments, the target nucleic acid sequence allows one to determine if the initial source of the target nucleic acid sequence is male or female (e.g., it is a sex specific marker). This can be achieved in a variety of ways, for example, by determining if the individual has two X chromosomes or an X and a Y chromosome. In some embodiments, this distinction can be determined by looking for specific sequence differences associated with either the X or Y chromosome. In some embodiments, this can be achieved by examining target nucleic acid sequences that are longer in one chromosome than the other. For example, by analyzing (and initially amplifying) amelogenin, which has a 6 base deletion in intron 1 for the X chromosome, one can determine if the sample includes only X chromosomes or both X and Y chromosomes. Thus, in some embodiments, the methods can be used to amplify sex-specific markers.

In some embodiments, initial sequences of the loci, of the standard stability primers (which can readily be modified as described herein), and of methods of their amplification and subsequent analysis of the results can be found in U.S. Pat. Nos: 7,008,771, 6,767,703, 6,479,235, and 6,221,598.

In some embodiments, the information obtained from amplifying and analyzing or characterizing a target nucleic acid sequence in a sample can be used in various applications, for example, in genetic mapping, linkage analysis, clinical diagnostics, or identity testing. In some embodiments, the information can be used to identify the source, or narrow down the possible sources, of the nucleic acid. In certain such embodiments, the information can be used, e.g., in forensic identification, paternity testing, DNA profiling, and related applications.

Personal identification tests can be performed on any sample that contains nucleic acid, such as bone, hair, blood, tissue and the like. DNA can be extracted from the sample and a primer set comprising a high stability primer to amplify a set of microsatellites used to amplify DNA in the presence of an inhibitor to generate a set of amplified fragments. In forensic testing, for example, the sample's microsatellite amplification pattern can be compared with a known sample from the presumptive victim (the presumed matching source) or can be compared to the pattern of amplified microsatellites derived from the presumptive victim's family members (*e.g.,* the mother and father) wherein the same set of microsatellites is amplified using high stability primer. The pattern of microsatellite amplification can be used to confirm or rule out the identity of the victim. In paternity testing, for example, the sample is generally from the child and the comparison is made to the microsatellite pattern from the presumptive father, and can comprise matching with the microsatellite pattern from the child's mother. The pattern of microsatellite amplification can be used to confirm or rule out the identity of the father. The panel can comprise microsatellites with a G+C content of 50% or less such as, for example, D3S1358; vWA; D16S539; D8S1179; D21S11; D18S51; D19S433; TH01; FGA; D7S820; D13S317; D5S818; CSF1PO; TPOX; hypoxanthine phosphoribosyltransferase; intestinal fatty acid-binding protein; recognition/surface antigen; c-fms proto-oncogene for CFS-1 receptor; tyrosine hydroxylase; pancreatic phospholipase A-2; coagulation factor XIII; aromatase cytochrome P-450; lipoprotein lipase; c-fes/fps proto-oncogene; and unknown fragment. The products can be examined by, for example, capillary electrophoresis coupled with GeneScan™ 310 analysis.

In some embodiments, a kit for a PCR reaction is provided. The kit comprises deoxynucleotide triphosphate; a high stability primer comprising at least one high stability nucleic acid analog; and DNA polymerase.

High stability primers suitable for the kit are described in detail above. In some embodiments, the kit can comprise one, two, three, four, five, six, seven, eight, nine, ten, eleven, twelve, thirteen, fourteen, fifteen, sixteen, seventeen, eighteen, nineteen, twenty or more high stability primers. In some embodiments, at least two primers can be present as a primer mix. In some embodiments, the primer mix comprises from about 5 pmoles/µL to 50 pmoles/µl each primer. In some embodiments, the primer mix comprises about 10, 15, 20, 25 or 30 pmoles/µL each primer. In some embodiments, the kit comprises at least one primer from a STR-specific primer set. In some embodiments, the kit comprises a STR-specific primer set.

In some embodiments, the kit further comprises a fluorescently labeled primer.

In some embodiments, the kit further comprises a container comprising an allelic ladder corresponding to sizes that are appropriate for comparison to a short tandem repeat analysis. The allelic ladder can be useful for analyzing STRs. In some embodiments, the kit can comprise an allelic ladder mix. In some embodiments, the allelic ladder mix can comprise allelic ladders for 1, 2, 3, 4, 5, 6, 7, 8, 9, 10, 11, 12, 13, 14, 15, 16 or more STR loci. In some embodiments, the allelic ladder can be a fluorescently labeled allelic ladder.

In some embodiments, the kit further comprises a DNA polymerase. The DNA polymerase can be thermostable. In some embodiments of the methods of the invention, the amplification comprises contacting said target nucleic acid with an enzyme having a polymerase activity. For example, the enzyme having polymerase activity can be selected from at least one of the following: DNA polymerase from Thermus aquaticus, Thermus thermophilus, other Thermus species, Bacillus species, Thermococcus species, Thermotoga species, and Pyrococcus species. For example, suitable polymerases comprise AmpliTaq Gold® DNA polymerase; AmpliTaq® DNA Polymerase; AmpliTaq® DNA Polymerase, Stoffel fragment; rTth DNA Polymerase; rTth DNA Polymerase XL; Bst DNA polymerase large fragment from Bacillus stearothermophilus; Vent and Vent Exo- from Thermococcus litoralis; Tma from Thermotoga maritima; Deep Vent and Deep Vent Exo- and Pfu from Pyrococcus; and mutants, variants and derivatives thereof.

In some embodiments, the kit further comprises MgCl₂. In some embodiments, the MgCl₂ provided with the kit can be present at a concentration of 10, 15, 20 or 25 mM. In some embodiments, the kit comprises sodium azide. In some embodiments, the kit comprises a 10x buffer solution comprising MgCl₂. In some embodiments, the kit comprises BSA. In some embodiments, the kit comprises a dNTP mix. In some embodiments, the dNTP mix can comprise 25 mM each nucleotide. In some embodiments of the invention, at least 0.5 mM each of dNTPs are used. In other embodiments, at least 1 mM dNTPs are used.

In some embodiments, the kit further comprises at least one control sample. In some embodiments, the kit can comprise a positive control, a negative control, an extraction blank control, or any combination thereof.

In some embodiments, the kit further comprises a mobility modifier. The mobility modifier can be polyethylene oxide, polyglycolic acid, polylactic acid, polypeptide, oligosaccharide, polyurethane, polyamide, polysulfonamide, polysulfoxide, polyphosphonate, or block copolymers thereof.

In some embodiments, the kit further comprises instructions. In some embodiments, the instructions can describe how to identify the presence of one or more target nucleic acids in the sample. In some embodiments, the instructions can describe how to identify the presence of a STR locus in a sample. In some embodiments, the instructions can describe how to identify the presence of a CODIS locus in a sample.

### Selection Of Standard Stability Primers And High Stability Primers

As will be appreciated by one of skill in the art, one way of determining possible sequences for high stability primers described herein is to start with a standard stability primer, which allows amplification of a loci of interest (*e.g.,* one that includes STRs) and to make permutations of it as described herein. In many embodiments, this can readily be achieved by taking known or published primer sequences used in STR and, more particularly, CODIS analysis, and using them as the initial starting template. Alternatively, additional high stability primer sequences can be determined by taking primer sized nucleic acid sequences from any of the presently disclosed loci (or other STR related loci of interest) and testing each primer sized section. Various nucleic acid analogs can be inserted into each position in the candidate high stability primer and the Tm of the resulting primer tested. Those candidate high stability primers that have an increase in Tm will be high stability primers. Each of the sequences surrounding each of the loci can be used to create numerous high stability primers. All possible standard stability primers for the presently disclosed STR and CODIS loci can readily be determined by one of skill in the art (*e.g.,* starting at a first nucleic acid in the locus, encompassing a section of nucleic acid of primer appropriate length (*e.g.,* 5-30 nucleic acids) to produce a first standard stability primer. One can move one nucleic acid position down the sequence to a new nucleic acid position (which can overlap with the previous primer sequence) and repeat the process as many times as one wishes to have primers.

In some embodiments, care should be used in selecting the sequence of primers used in the multiplex reaction. Inappropriate selection of primers can produce several undesirable effects such as lack of amplification, amplification at multiple sites, primer dimer formation, undesirable interaction of primer sequences from different loci, production of alleles from one locus which overlap with alleles from another, or the need for amplification conditions or protocols for the different loci which are incompatible in a multiplex. Standard stability primers can be selected according to the following selection process.

In some embodiments, the primers are developed and selected for use in the multiplex systems by employing a re-iterative process of selecting primer sequences, mixing the primers for co-amplification of the selected loci, co-amplifying the loci, then separating and detecting the amplified products. Initially, this process often produces the amplified alleles in an imbalanced fashion (i.e., higher product yield for some loci than for others) and may also generate amplification products which do not represent the alleles themselves.

To eliminate such extra fragments from the multiplex systems, individual primers from the total set are used with primers from the same or other loci to identify which primers contribute to the amplification of the extra fragments. Once two primers which generate one or more of the fragments are identified, one or both primers are modified and retested, either in a pair alone or in the multiplex system (or a subset of the multiplex system). This process is repeated until evaluation of the products yields amplified alleles with no or an acceptable level of extra amplification products in the multiplex system.

On occasion, extra amplification products can be eliminated by labeling the opposite primer in a primer pair. This change reveals the products of the opposing primer in the detection step. This newly labeled primer can amplify the true alleles with greater fidelity than the previously labeled primer generating the true alleles as a greater proportion of the total amplification product.

The determination of primer concentration can be performed either before or after selection of the final primer sequences, but is preferably performed after that selection. Generally, increasing primer concentration for any particular locus increases the amount of product generated for that locus. However, this is also a re-iterative process because increasing yield for one locus may decrease it for one or more other loci. Furthermore, primers may interact directly affecting yield of the other loci. Linear increases in primer concentration do not necessarily produce linear increases in product yield for the corresponding locus.

Locus to locus balance can also be affected by a number of parameters of the amplification protocol such as the amount of template used, the number of cycles of amplification, the annealing temperature of the thermal cycling protocol, and the inclusion or exclusion of an extra extension step at the end of the cycling process. Absolutely even balance across all alleles and loci is generally not achieved nor is it necessary to produce useful allele information.

The process of multiplex system development can also be a re-iterative process in another sense. That is, it is possible, first, to develop a multiplex system for a small number of loci, this system being free or nearly free of extra fragments from amplification. Primers of this system may be combined with primers for one or more additional loci. This expanded primer combination may or may not produce extra fragments from amplification. In turn, new primers can be introduced and evaluated.

One or more of the re-iterative selection processes described above are repeated until a complete set of primers is identified which can be used to co-amplify the loci selected for co-amplification as described herein. It is understood that many different sets of primers can be developed to amplify a particular set of loci.

Synthesis of the standard hybridization primers can be conducted using any standard procedure for oligonucleotide synthesis known to those skilled in the art.

As will be appreciated by one of skill in the art, the above method for determining standard stability primers can be modified for determining high stability primers by employing primers that comprise the high stability nucleic acid analogs and selecting between them in a similar manner. Of course, the final Tm of the candidate high stability primer can be verified as well.

### Preparation of DNA Samples

Samples can be prepared for use in the method using any method of DNA preparation which is compatible with the amplification of DNA. Many such methods are known by those skilled in the art. Examples include, but are not limited to DNA purification by phenol extraction (Sambrook, J., et al. (1989) Molecular Cloning: A Laboratory Manual, Second Edition, Cold Spring Harbor Laboratory Press, Cold Spring Harbor, N.Y., pp. 9.14 9.19), and partial purification by salt precipitation (Miller, S. et al. (1988) Nucl. Acids Res. 16:1215) or chelex (Walsh et al., (1991) BioTechniques 10:506 513, Comey, et al., (1994) Forensic Sci. 39:1254) and the release of unpurified material using untreated blood (Burckhardt, J. (1994) PCR Methods and Applications 3:239 243, McCabe, Edward R. B.,(1991) PCR Methods and Applications 1:99 106, Nordvag, Bjorn-Yngvar (1992) BioTechniques 12:4 pp. 490 492).

When the at least one sample to be analyzed using the method of this invention comprises human genomic DNA, the DNA is can be prepared from tissue, selected from the group consisting of blood, semen, vaginal cells, hair, saliva, urine, bone, buccal samples, amniotic fluid containing placental cells or fetal cells, chorionic villus, and mixtures of any of the tissues listed above.

Optionally, DNA concentrations can be measured prior to use in the method using any standard method of DNA quantification known to those skilled in the art. In such cases, the DNA concentration can be determined by spectrophotometric measurement as described by Sambrook, J., et al. (1989), supra, Appendix E.5, or fluorometrically using a measurement technique such as that described by Brunk C. F., et al. (1979), Anal Biochem 92: 497 500. The DNA concentration can be measured by comparison of the amount of hybridization of DNA standards with a human-specific probe such as that described by Waye, J. S., et al. (1991) "Sensitive and specific quantification of human genomic deoxyribonucleic acid (DNA) in forensic science specimens: casework examples," J. Forensic Sci., 36:1198 1203. Use of too much template DNA in the amplification reactions can produce artifacts which appear as extra bands which do not represent true alleles. In some embodiments, quantitative techniques, such as TAQMAN® PCR can be employed. In some embodiments, any of the amplifying techniques discussed above can be used. In some embodiments, real-time PCR analysis is used. In some embodiments, SYBR Green dye is used In some embodiments, a 5' nuclease process is employed.

As will be appreciated by one of skill in the art, in some embodiments, the use of a high stability primer can allow for some of the above steps to be removed, as the purification of the sample can be less critical.

### Amplification of DNA in Multiplex Amplifications

Once a sample is prepared, the targeted loci can be co-amplified in a multiplex amplification step. Any one of a number of different amplification methods can be used to amplify the loci, including, but not limited to, polymerase chain reaction (PCR) (Saiki, R. K., et al. (1985), Science 230: 1350 1354), transcription based amplification (Kwoh, D. Y., and Kwoh, T. J. (1990), American Biotechnology Laboratory, October, 1990) and strand displacement amplification (SDA) (Walker, G. T., et al. (1992) Proc. Natl. Acad. Sci., U.S.A. 89: 392 396). In some embodiments, the DNA sample is subjected to PCR amplification using high stability primer pairs specific to each locus in the set.

In some embodiments, at least one high stability primer for each locus can be covalently attached to a dye label, more preferably a fluorescent dye label. The high stability primers and dyes attached thereto can be selected for the multiplex amplification reaction, such that alleles amplified using primers for each locus labeled with one color do not overlap the alleles of the other loci in the set co-amplified therein using high stability primers labeled with the same color, when the alleles are separated, preferably, by gel or capillary electrophoresis.

In some embodiments, at least one high stability primer for each locus co-amplified in the multiplex reaction is labeled with a fluorescent label prior to use in the reaction. Fluorescent labels suitable for attachment to primers for use in the present invention are commercially available. See, *e.g*. fluorescein and carboxy-tetramethylrhodamine labels and their chemical derivatives from PE Biosystems and Molecular Probes. In some embodiments, at least three different labels are used to label the different high stability primers used in the multiplex amplification reaction. When a size marker is included to evaluate the multiplex reaction, the primers used to prepare the size marker can be labeled with a different label from the primers used to amplify the loci of interest in the reaction.

In some embodiments, the sequences of the locus-specific high stability primers used include a number of nucleotides which, under the conditions used in the hybridization, are sufficient to hybridize with an allele of the locus to be amplified and to be essentially free from amplification of alleles of other loci. Reference is made to U.S. Pat. No. 5,192,659 to Simons.

### Separation and Detection of DNA Fragments

Once a set of amplified alleles is produced from the multiplex amplification step, the amplified alleles are evaluated. The evaluation step of this method can be accomplished by any one of a number of different means, some of which are described below.

Electrophoresis is can be used to separate the products of the multiplex amplification reaction, as can capillary electrophoresis (see, e.g., Buel, Eric et al. (1998), Journal of Forensic Sciences; 43:(1) pp. 164 170) or denaturing polyacrylamide gel electrophoresis (see, e.g., Sambrook, J. et al. (1989) In Molecular Cloning-A Laboratory Manual, 2nd edition, Cold Spring Harbor Laboratory Press, pp. 13.45 1 3.57). Gel preparation and electrophoresis procedures and conditions for suitable for use in the evaluating step of the method are illustrated in the Examples, below. Separation of DNA fragments in a denaturing polyacrylamide gel and in capillary electrophoresis occurs based primarily on fragment size, but can be adjusted by the use of mobility modifiers.

Once the amplified alleles are separated, the alleles and any other DNA in the gel or capillary (e.g., DNA size markers or an allelic ladder) can then be visualized and analyzed. Visualization of the DNA in the gel can be accomplished using any one of a number of techniques, including silver staining or reporters such as radioisotopes, fluorescers, chemiluminescers and enzymes in combination with detectable substrates. In some embodiments, the method for detection of multiplexes containing thirteen or more loci comprises fluorescence (*see, e.g.,* Schumm, J. W. et al. in Proceedings from the Eighth International Symposium on Human Identification, (pub. 1998 by Promega Corporation), pp. 78 84; Buel, Eric et al. (1998), supra.), wherein high stability primers for each locus in the multiplexing reaction is followed by detection of the labeled products employing a fluorometric detector.

The alleles present in the DNA sample can be determined by comparison to a size standard such as a DNA marker or a locus-specific allelic ladder to determine the alleles present at each locus within the sample. In some embodiments, the size of the marker for evaluation of a multiplex amplification containing two or more polymorphic STR loci includes of a combination of allelic ladders for each of the loci being evaluated. See, e.g., Puers, Christoph et al., (1993) Am J. Hum Genet. 53:953 958, Puers, Christoph, et al. (1994) Genomics 23:260 264. See also, U.S. Pat. No's 5,599,666; 5,674,686; and 5,783,406 for descriptions of allelic ladders suitable for use in the detection of STR loci, and methods of ladder construction disclosed therein.

Following the construction of allelic ladders for individual loci, these can be mixed and loaded for gel electrophoresis at the same time as the loading of amplified samples occurs. Each allelic ladder co-migrates with alleles in the sample from the corresponding locus.

The products of the multiplex reactions of the present invention can be evaluated using an infernal lane standard, a specialized type of size marker configured to run in the same lane of a polyacrylamide gel or same capillary. The internal lane standard can include a series of fragments of known length. The internal lane standard more preferably is labeled with a fluorescent dye which is distinguishable from other dyes in the amplification reaction.

Following construction of the internal lane standard, this standard can also be mixed with amplified sample or allelic ladders and loaded for electrophoresis for comparison of migration in different lanes of gel electrophoresis or different capillaries of capillary electrophoresis. Variation in the migration of the internal lane standard indicates variation in the performance of the separation medium. Quantitation of this difference and correlation with the allelic ladders allows correction in the size determination of alleles in unknown samples.

### Optional Detection Technique: Fluorescent Detection

In some embodiments, fluorescent detection is used to evaluate the amplified alleles in the mixture produced by the multiplex amplification reaction using the high stability primer(s). Below is a brief summary of how that method of detection can be practiced.

With the advent of automated fluorescent imaging, faster detection and analysis of multiplex amplification products can be achieved. For fluorescent analysis, one fluorescent labeled high stability primer can be included in the amplification of each locus. Fluorescent labeled high stability primers suited for use can include the fluorescein-labeled (FL-), carboxy-tetramethylrhodamine-labeled (TMR-), and 5,6-carboxyrhodamine 6G-labeled (R6G) high stability primers. Separation of the amplified fragments produced using such labeled high stability primers can be achieved preferably by slab gel electrophoresis or capillary electrophoresis. The resulting separated fragments can be analyzed using fluorescence detection equipment such as an ABI PRISM® 310 Genetic Analyzer, an ABI PRISM® 377 DNA Sequencer (Applied Biosystems Division, Perkin Elmer, Foster City, Calif.), or a Hitachi FMBIO® II Fluorescent Scanner (Hitachi Software Engineering America, Ltd. South San Francisco, Calif.).

In some embodiments, one or both of each pair of high stability primers used in the multiplex amplification reaction has a fluorescent label attached thereto, and as a result, the amplified alleles produced from the amplification reaction are fluorescently labeled. In this embodiment, the amplified alleles are subsequently separated by capillary electrophoresis and the separated alleles visualized and analyzed using a fluorescent image analyzer.

Fluorescent detection is can be advantageous over radioactive methods of labeling and detection, because it does not require the use of radioactive materials, and all the regulatory and safety problems which accompany the use of such materials.

Fluorescent detection employing labeled high stability primers can also be used over other non-radioactive methods of detection, such as silver staining, because fluorescent methods of detection generally reveal fewer amplification artifacts than silver staining. The smaller number of artifacts are due, in part, to the fact that only amplified strands of DNA with labels attached are detected in fluorescent detection, while both strands of every amplified allele of DNA produced from the multiplex amplification reaction is stained and detected using the silver staining method of detection.

### Examples

Aspects of the present teachings can be further understood in light of the following examples, which should not be construed as limiting the scope of the present teachings in any way.

### Example 1

This example illustrates an assay to test for the presence of a PCR inhibitor in a sample (or to determine if a substance is an inhibitor). As noted above, the identification of the presence of an inhibitor in a sample is an optional step.

In this example, a sample comprising a target nucleic acid sequence and a possible PCR inhibitor is provided. This is combined with a set of standard stability primers for amplifying the target nucleic acid sequence. A control solution comprising the same amount of target nucleic acid sequence and set of primers as the sample reaction is set up in a separate container. Amplification reactions on the sample and control are performed. The amplification results from the sample and the control reactions are analyzed and compared. The absence of a PCR product in the sample reaction indicates the presence of an inhibitor in the sample. In the alternative, the presence of a smaller amount of PCR product in the sample reaction as compared to the amount of PCR product in the control reaction is indicative of an inhibitor in the sample.

### Example 2

This example illustrates an assay to test for high stability primers comprising a high stability nucleic acid analog.

In this example, a primer that only comprises naturally occurring nucleic acids (*e.g*., a standard stability primer) is provided. A candidate high stability primer is created having the same sequence as the standard stability primer, but using a comparable (*e.g*., it base pairs with the same selectivity) high stability nucleic acid analog in the place of at least one of the naturally occurring nucleic acids. The melting point temperature of the test primer is tested and compared to the melting point temperature of the standard stability primer. A higher melting point is indicative of a more stable primer. If the melting point of the candidate high stability primer is higher than the melting point of the standard stability primer, this is an indication that the candidate high stability primer is more stable than the standard stability primer.

In the alternative, a candidate high stability primer is created having the same sequence as the standard stability primer using two high stability nucleic acid analogs in the place of two nucleic acids. The melting point temperature of the candidate high stability primer is tested and compared to the melting point temperature of the standard stability primer. If the melting point of the candidate high stability primer is higher than the melting point of the standard stability primer, this is an indication that the candidate high stability primer is more stable than the standard stability primer.

In the alternative, a candidate high stability primer is created having the same sequence as the standard stability primer using three high stability nucleic acid analogs in the place of three nucleic acids. The melting point temperature of the candidate high stability primer is tested and compared to the melting point temperature of the standard stability primer. A higher melting point is indicative of a more stable primer. If the melting point of the candidate high stability primer is higher than the melting point of the standard stability primer, this is an indication that the candidate high stability primer is more stable than the standard stability primer.

### Example 3

This example illustrates amplification of a target nucleic acid sequence in a sample comprising a PCR inhibitor.

A sample comprising a target nucleic acid sequence and a PCR inhibitor is provided. The target nucleic acid sequence is combined with a primer set comprising a high stability primer. The high stability primer comprises a high stability nucleic acid analog. An amplification reaction on the sample (or at least including the target nucleic acid sequence) is performed, and the target nucleic acid sequence is amplified via the high stability primer. The target nucleic acid sequence will be amplified with a greater degree of efficiency, even in the presence of the PCR inhibitor.

In the alternative, a sample comprising a target nucleic acid sequence and two or three PCR inhibitors is provided. The target nucleic acid sequence is combined with a primer set comprising a high stability primer. The high stability primer comprises a high stability nucleic acid analog. An amplification reaction on the sample is performed, and the target nucleic acid sequence is amplified via the high stability primer. The target nucleic acid sequence will be amplified with a greater degree of efficiency, even in the presence of the PCR inhibitors.

In the alternative, a sample from a crime scene is provided. The sample comprising a target nucleic acid sequence and a PCR inhibitor. The target nucleic acid sequence is combined with a primer set comprising a high stability primer comprising a high stability nucleic acid analog. An amplification reaction on the sample is performed, and the target nucleic acid sequence is amplified via the high stability primer. The target nucleic acid sequence will be amplified with a greater degree of efficiency, even in the presence of the PCR inhibitor.

### Example 4

This example illustrates amplification of a target nucleic acid sequence in a sample comprising a PCR inhibitor.

A sample comprising a target nucleic acid sequence and a PCR inhibitor is provided. The target nucleic acid sequence is combined with a primer set comprising a high stability primer. The primer set can be capable of amplifying a CODIS nucleic acid sequence or another STR containing locus. The high stability primer comprises two or three high stability nucleic acid analogs. An amplification reaction on the sample is performed, and the target nucleic acid sequence is amplified via the high stability primer.

### Example 5

This example illustrates amplification of a target nucleic acid sequence in a sample comprising a PCR inhibitor.

A sample comprising a target nucleic acid sequence and a PCR inhibitor is provided. The target nucleic acid sequence is combined with a primer set comprising two (or three) high stability primers. Each primer comprises a high stability nucleic acid analog. An amplification reaction on the sample is performed, and the target nucleic acid sequence is amplified via the high stability primers. The target nucleic acid sequence will be amplified to a greater extent than if just a comparable standard stability primer had been used.

### Example 6

This example illustrates amplification of an STR locus in a sample comprising nucleic acid and a PCR inhibitor.

A sample comprising a target nucleic acid sequence and a PCR inhibitor is provided. The sample comprising nucleic acid and a PCR inhibitor is combined with an STR-specific primer set comprising a high stability primer. An amplification reaction on the sample is performed, and the STR locus is amplified via the high stability primer.

### Example 7

This example illustrates the amplification of a CODIS locus in a sample comprising a PCR inhibitor.

A sample comprising a target nucleic acid sequence and I suspected of having a PCR inhibitor is provided. The target nucleic acid sequence is combined with a CODIS-specific primer set comprising at least one high stability primer. An amplification reaction on the sample is performed, and the CODIS locus is amplified via the high stability primer.

### Example 8

This example illustrates amplification of one or more loci from D8S1179, D18S51, D21S11, FGA, TH01, vWA, and Amelogenin in the presence of a PCR inhibitor.

A sample comprising a target nucleic acid sequence and a PCR inhibitor is provided. The sample comprising the target nucleic acid sequence and a PCR inhibitor is combined with a primer set comprising at least one high stability primer specific for each of the D8S 1179, D18S51, D21S11, FGA, TH01, vWA, and Amelogenin loci. An amplification reaction on the sample is performed, and the desired loci are amplified via the high stability primers.

### Example 9

This example illustrates amplification of one or more loci from D8S1179, D18S51, D21S11, FGA, TH01, vWA, D2S1338, D3S1358, D16S539, D19S433, SE33 and Amelogenin in the presence of a PCR inhibitor.

A sample comprising a target nucleic acid sequence and a PCR inhibitor is provided. The sample comprising the target nucleic acid sequence and the PCR inhibitor is combined with a primer set comprising at least one high stability primer specific for each of the D8S1179, D18S51, D21S11, FGA, TH01, vWA, D2S1338, D3S1358, D16S539, D19S433, SE33 and Amelogenin loci. An amplification reaction on the sample is performed, and the desired loci are amplified via the high stability primers.

### Example 10

This example illustrates amplification of one or more loci from D8S1179, D21S11, D7S820, CSF1PO, D3S1358, TH01, D13S317, D16S539, D2S1338, D19S433, vWA, TPOX, D18S51, D5S818 and FGA.

A sample comprising a target nucleic acid sequence and a PCR inhibitor is provided. The sample comprising the target nucleic acid sequence and the PCR inhibitor is combined with a primer set comprising at least one high stability primer specific for each of the D8S1179, D21S11, D7S820, CSF1PO, D3S1358, TH01, D13S317, D16S539, D2S1338, D19S433, vWA, TPOX, D18S51, D5S818 and FGA loci. An amplification reaction on the sample is performed, and the desired loci are amplified via the high stability primers.

### Example 11

This example illustrates amplification of one or more loci from Penta E, D18S51, D21S11, TH01, D3S1358, FGA, TPOX, D8S1179, vWA, Amelogenin, and Penta D, CSF1PO, D16S539, D7S820, D13S317, and D5S818.

A sample comprising a target nucleic acid sequence and a PCR inhibitor is provided. The sample comprising the target nucleic acid sequence and a PCR inhibitor is combined with primer set comprising at least one high stability primer specific for each of the Penta E, D18S51, D21S11, TH01, D3S1358, FGA, TPOX, D8S1179, vWA, Amelogenin, and Penta D, CSF1PO, D16S539, D7S820, D13S317, and D5S818 loci. An amplification reaction on the sample is performed, and the desired loci are amplified via the high stability primers.

### Example 12

This example illustrates amplification of one or more loci from DYS19, DYS385a/b, DYS389I/II, DYS390, DYS391, DYS392, DYS393, DYS437, DYS438 and DYS439.

A sample comprising a target nucleic acid sequence and a PCR inhibitor is provided. The sample comprising the target nucleic acid sequence and the PCR inhibitor is combined with an primer set comprising at least one high stability primer specific for each of the DYS19, DYS385a/b, DYS389I/II, DYS390, DYS391, DYS392, DYS393, DYS437, DYS438 and DYS439. An amplification reaction on the sample is performed, and the desired loci are amplified via the high stability primers.

### Example 13

This Example demonstrates how one can make various embodiments of the high stability primers. As will be appreciated by one of skill in the art, the nucleic acid sequences associated with the following loci are known: TH01, TPOX, CSF1PO, vWA, FGA, D3S1358, D5S818, D7S820, D13S317, D16S539, D8S1179, D18S51, D21S11, D2S1338, D3S1539, D4S2368, D9S930, D10S1239, D14S118, D14S548, D14S562, D16S490, D16S753, D17S1298, D17S1299, D19S253, D19S433, D20S481, D22S683, HUMCSF1PO, HUMTPOX, HUMTH01, HUMF13AO1, HUMBFXIII, HUMLIPOL, HUMvWFA31. One selects a section of one or more of the nucleic acid sequences (from the loci above) as a binding site for an amplification primer (any sequence can suffice, as long as it allows amplification of the relevant STR). The nucleic acid section will be long enough to allow a primer to bind to it as desired (*e*.*g*., to function as a primer, 5-30 nucleic acids in length). This section will be used to generate a standard stability primer (which will hybridize to the initial sequence selected above). The standard stability primer will be the complementary sequence to the selected section.

A comparable candidate high stability primer is then generated to the standard stability primer. The candidate high stability primer will be identical to the standard stability primer, apart from one or more high stability nucleic acid substitutions that are present in the candidate high stability primer. The high stability nucleic acid substitutions will be selected so that they have the same base pairing selectivity properties that the replaced nucleic acid(s) possessed. This comparable replacement can continue as many times as desired (to produce as many primer sequences as desired). The "replacement" need not actually be a physical replacement of one natural nucleic acid with a nucleic acid analog, rather, a new primer can be synthesized which, apart from the nucleic acid analog, is identical to the standard stability primer.

Once a candidate high stability primer(s) is generated, it can be tested for its amplification ability in the presence of an amplification inhibitor. This can be achieved by adding a known amount of the initial target nucleic acid sequence (one of the loci noted above), adding a known amount of an inhibitor (e.g., humic acid, bile salt, other salt, complex polysaccharides, collagen, heme, melanin, eumelanin, myoglobin, polysaccharides, proteinases, calcium ions, urea, hemoglobin, lactoferrin, immunoglobulin G, indigo dye, hemoglobin, fulvic acid, divalent cations, chelating molecules, enzyme, proteins, complex polysaccharides, EDTA, EGTA, DNAse, and collagen), and adding each of the candidate high stability primers. The amount of amplification from using the candidate high stability primer can be compared to the amount of amplification that occurs when the standard stability primer is used. Those candidate primers that exhibit a higher degree of amplification ability compared to the standard stability primer will be high stability primers.

In an alternative embodiment, the high stability nucleic acid analog is selected from one of the following: PNA, LNA, a 2'-O-Methyl nucleic acid, a 2'-O-Alkyl nucleic acid, a 2'-fluoro nucleic acid, a nucleic acid including a phosphorothioate linkage, or any combination thereof.

Given this example, the knowledge of one of skill in the art, and the teachings disclosed herein, one of skill in the art will be able to prepare a high stability primer for any section of any of the above noted loci.

### Example 14

This example demonstrates how one of skill in the art can identify an amplification inhibitor. First, one obtains a known target nucleic acid sequence and a standard stability primer for that target nucleic acid sequence. One combines them in a buffer solution for a standard PCR reaction. One divides the solution into two parts. To the first part, one adds a candidate PCR inhibitor to the PCR reaction. No inhibitor is added to the second part. Both parts are PCR amplified in parallel and the amount of product resulting in each part is compared. If the first part results in less amplified product, then the candidate PCR inhibitor is a PCR inhibitor.

### Example 15

This example demonstrates how one can use the high stability primers to identify a target individual. A sample comprising a target nucleic acid sequence and a PCR inhibitor is provided. The sample is combined with a primer set comprising at least one high stability primer specific for at least 5 of the following loci: D8S1179, D18S51, D21S11, FGA, TH01, vWA, D2S1338, D3S1358, D16S539, D19S433, SE33 and Amelogenin. An amplification reaction on the sample is performed, and the desired loci are amplified via the high stability primers.

The amplified sequences are then examined, allowing the characterization of the short tandem repeats in the above loci (*e.g.,* identifying how many short tandem repeats are present for each loci). These results are then compared to the STR characteristics of the target individual. If the STRs are the same, then the target individual is considered to be a match to the target nucleic acid sequence.

### Example 16

This example demonstrates one method of amplifying a target nucleic acid sequence by using a high stability primer. One first provides a forensic sample that is suspected of comprising a target nucleic acid sequence. The forensic sample is treated so as to place the target nucleic acid in a buffer for subsequent amplification and to allow the release the target nucleic acid from cells or cellular components that can be present in the sample.

Following this, one then combines at least one high stability primer with the target nucleic acid sequence. The high stability primer specifically hybridizes to the target nucleic acid sequence in a manner to allow amplification of the target nucleic acid sequence. Following this, one amplifies the target nucleic acid sequence via TAQMAN® PCR, thereby amplifying a target nucleic acid sequence.

Optionally, one can characterize the amplified target nucleic acid sequence and then compare that characterization to an individual's profile to determine if the individual is or is not a match to the target nucleic acid sequence and the sample in general.

### Example 17

This example illustrates an enhanced performance of high stability primers by replacement of existing oligonucleotides with LNA-containing oligonucleotides. The example also provides general guidance for how one of skill in the art could test various primers for the various CODIS related sequences and determine where and how many high stability nucleic acid analogs should be placed in the various primers. A variety of amelogenin-LNA-containing oligonucleotides were prepared. Each primer included at least one LNA, and some included two LNAs.

The amelogenin-LNA primers were tested for amplification ability in controlled conditions with and without humic acid (a PCR inhibitor) at 40 ng/µL. The results were compared to the amplification ability of a control sequence that did not include LNA. At 40 ng/microliter, partial inhibition of the PCR was obtained during the amplification of 1 ng of male DNA with the control primer. In contrast, some of the LNA containing primers exhibited substantially less inhibition.

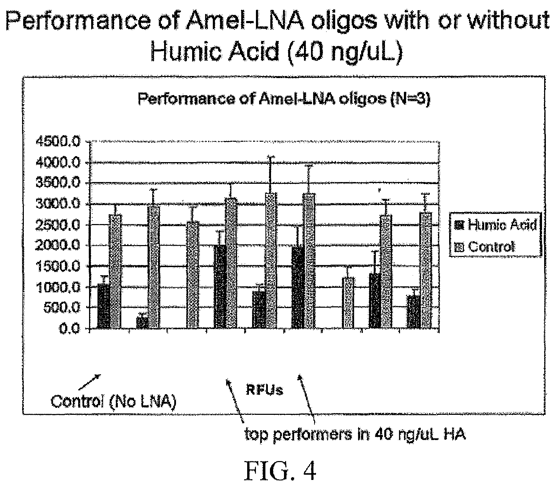

Moreover, some of the amelogenin LNA-oligonucleotides overcame humic acid inhibition at 40 ng/uL (**FIG. 4**). In contrast, the control oligonucleotide failed to amplify under identical conditions.

### Example 1

This example demonstrates how a sex specific marker can be amplified to determine if the source of a target nucleic acid sequence is male or female.

A sample comprising a target nucleic acid sequence and a PCR inhibitor is provided. The sample comprising the target nucleic acid sequence and a PCR inhibitor is combined with a primer set comprising at least one high stability primer specific the Amelogenin loci. An amplification reaction on the sample is performed, and the desired loci are amplified via the high stability primers.

The amplified product is then examined. If the amplified product comprises only nucleic acid sequences having a 6 base deletion in intron 1, then the subject is female. If the amplified product comprises a mixture of sequencing having the 6 base deletion in intron 1 and not having the deletion in intron 1, then the subject is male.

It is to be understood that both the foregoing general description and the detailed description are exemplary and explanatory only and are not restrictive of the invention, as claimed. In this application, the use of the singular includes the plural unless specifically stated otherwise. In this application, the word "a" or "an" means "at least one" unless specifically stated otherwise. In this application, the use of "or" means "and/or" unless stated otherwise. Furthermore, the use of the term "including," as well as other forms, such as "includes" and "included," is not limiting. Also, terms such as "element" or "component" encompass both elements or components comprising one unit and elements or components that comprise more than one unit unless specifically stated otherwise.

The section headings used herein are for organizational purposes only and are not to be construed as limiting the described subject matter in any way.

It will be appreciated that there is an implied "about" prior to the temperatures, concentrations, times, etc. discussed in the present teachings, such that slight and insubstantial deviations are within the scope of the present teachings herein. For example, "a primer" means that more than one primer can, but need not, be present; for example but without limitation, one or more copies of a particular primer species, as well as one or more versions of a particular primer type, for example but not limited to, a multiplicity of different forward primers. Also, the use of "comprise", "comprises", "comprising", "contain", "contains", "containing", "include", "includes", and "including" are not intended to be limiting. It is to be understood that both the foregoing general description and detailed description are exemplary and explanatory only and are not restrictive of the invention.

## Claims

1. A method comprising
contacting a sample comprising a nucleic acid and a PCR inhibitor that is humic acid with a primer, wherein the primer comprises a single high stability nucleic acid analog, wherein the single high stability nucleic acid analog is an LNA, and wherein the primer exhibits a higher degree of amplification ability in the presence of humic acid than a standard primer not comprising said single LNA; and
performing a PCR amplification reaction on the sample, wherein a target nucleic acid sequence is amplified by PCR with the primer comprising said single LNA.

2. The method of Claim 1, wherein the PCR amplification with the primer amplifies a nucleic acid sequence from at least one locus selected from the group consisting of: CSF1PO, FGA, TH01, TPOX, vWA, D3S1358, DSS818, D7S820, DSS1179, D13S317, D16S539, D18S51, D21S11, D19S433, D2S1338, and amelogenin.

3. The method of claim 1, wherein the primer comprises a sequence that allows the amplification of a short tandem repeat.

4. The method of claim 1, wherein the primer further comprises a mobility modifier.

5. The method of claim 4, wherein the mobility modifier is selected from the group consisting of: polyethylene oxide, polyglycolic acid, polylactic acid, polypeptide, oligosaccharide, polyurethane, polyamide, polysulfonamide, polysulfoxide, polyphosphonate, and block copolymers thereof.

6. Use of a kit for performing a method of claims 1-5, the kit comprising:
deoxynucleotide triphosphate;
a fluorescently labeled primer;
a high stability primer comprising a single high stability nucleic acid analog, wherein the single high stability nucleic acid analog is LNA, and wherein the primer exhibits a higher degree of amplification ability in the presence of humic acid than a standard primer not comprising said single LNA;
a DNA polymerase; and
optionally an allelic ladder corresponding to sizes that are appropriate for comparison to a short tandem repeat analysis.

## Patentansprüche

1. Verfahren, umfassend:
Inkontaktbringen einer Probe, umfassend eine Nukleinsäure und einen PCR-Inhibitor, der Huminsäure ist, mit einem Primer, wobei der Primer ein einzelnes, hochstabiles Nukleinsäureanalog ist, wobei das einzelne, hochstabile Nukleinsäureanalog eine LNA ist, und wobei der Primer ein höheres Maß an Amplifikationsfähigkeit in Gegenwart der Huminsäure aufweist als ein Standardprimer ohne die einzelne LNA; und
Durchführen einer PCR-Amplifikationsreaktion an der Probe, wobei eine Target-Nukleinsäuresequenz mittels PCR mit dem Primer, umfassend die einzelne LNA, amplifiziert wird.

2. Verfahren nach Anspruch 1, wobei die PCR-Amplifikation mit dem Primer eine Nukleinsäuresequenz von mindestens einem Locus amplifiziert, der aus der Gruppe ausgewählt ist, die aus Folgendem besteht: CSF1PO, FGA, TH01, TPOX, vWA, D3S1358, DSS818, D7S820, DSS1179, D13S317, D16S539, D18S51, D21S11, D19S433, D2S1338 und Amelogenin.

3. Verfahren nach Anspruch 1, wobei der Primer eine Sequenz umfasst, die die Amplifikation eines Short Tandem Repeats ermöglicht.

4. Verfahren nach Anspruch 1, wobei der Primer weiterhin einen Mobilitätsmodifizierer umfasst.

5. Verfahren nach Anspruch 4, wobei der Mobilitätsmodifizierer aus der Gruppe ausgewählt ist, die aus Folgendem besteht: Polyethylenoxid, Polyglycolsäure, Polymilchsäure, Polypeptid, Oligosaccharid, Polyurethan, Polyamid, Polysulfonamid, Polysulfoxid, Polyphosphonat und Blockcopolymere davon.

6. Verwendung eines Kits zur Durchführung eines Verfahrens der Ansprüche 1 bis 5, wobei der Kit Folgendes umfasst:
Desoxynukleotidtriphosphate;
einen fluoreszenzmarkierten Primer;
einen hochstabilen Primer, umfassend ein einzelnes, hochstabiles Nukleinsäureanalog, wobei das einzelne, hochstabile Nukleinsäureanalog eine LNA ist, und wobei der Primer ein höheres Maß an Amplifikationsfähigkeit in Gegenwart von Huminsäure aufweist als ein Standardprimer ohne die einzelne LNA;
eine DNA-Polymerase; und
optional eine Allel-Leiter, die Größen entspricht, die zum Vergleich mit einer Short Tandem Repeat-Analyse geeignet sind.

## Revendications

1. Procédé comprenant :
la mise en contact d'un échantillon contenant un acide nucléique et un inhibiteur de PCR qui est de l'acide humique avec une amorce, où l'amorce comprend un unique analogue d'acide nucléique de grande stabilité, où l'unique analogue d'acide nucléique de grande stabilité est un acide nucléique bloqué, et où l'amorce présente un plus haut degré de capacité d'amplification en présence d'acide humique qu'une amorce standard ne comprenant pas ledit unique acide nucléique bloqué ; et
la réalisation d'une réaction d'amplification PCR sur l'échantillon, où une séquence cible d'acide nucléique est amplifiée par PCR avec l'amorce comprenant ledit unique acide nucléique bloqué.

2. Procédé selon la revendication 1, dans lequel l'amplification PCR avec l'amorce amplifie une séquence d'acide nucléique depuis au moins un locus choisi dans le groupe constitué de: CSF1PO, FGA, TH01, TPOX, Vwa, D3S1358, DSS818, D7S820, DSS1179, S13S317, D16S539, D18S51, D21S11, D9S433, D2S1338 et amélogénine.

3. Procédé selon la revendication 1, dans lequel l'amorce comprend une séquence qui permet l'amplification d'une courte répétition en tandem.

4. Procédé selon la revendication 1, dans lequel l'amorce comprend, en outre, un modificateur de mobilité.

5. Procédé selon la revendication 4, dans lequel le modificateur de mobilité est choisi dans le groupe constitué de : polyoxyde d'éthylène, polyacide glycolique, polyacide lactique, polypeptide, oligosaccharide, polyuréthane, polyamide, polysulfonamide, polysulfoxyde, polyphosphonate et des copolymères blocs de ceux-ci.

6. Utilisation d'un kit pour effectuer un procédé selon l'une quelconque des revendications 1 à 5, le kit comprenant:
un désoxynucléotide triphosphate ;
une amorce marquée par fluorescence;
une amorce de grande stabilité comprenant un unique analogue d'acide nucléique de grande stabilité, où l'unique analogue d'acide nucléique de grande stabilité est un acide nucléique bloqué, et où l'amorce présente un plus haut degré de capacité d'amplification en présence d'acide humique qu'une amorce standard ne comprenant pas ledit unique acide nucléique bloqué ;
une ADN polymérase ; et
facultativement, une échelle allélique correspondant aux dimensions appropriées pour comparaison à une analyse de courte répétition en tandem.
